# FASCICULE DE BREVET EUROPEEN

(11) **EP 1 335 201 B1**
(45) Date de publication et mention de la délivrance du brevet: **10.10.2012**
(21) Numéro de dépôt: 03290302.3
(22) Date de dépôt: 06.02.2003
(51) Int. Cl.: G01N 27/447, G01N 33/561

(54) **Masque pour le dépôt et l'étalement de réactifs sur un support d'analyse**
Maske zur Beschichtung und zum Bestreuen von Reaktionsmitteln auf einem Analysenträger
Mask for depositing and spreading reactant on an analysis support

(30) Priorité: 06.02.2002 FR 0201433
(43) Date de publication de la demande: 13.08.2003
(73) Titulaire: LABORATOIRES SEBIA, 92130 Issy-Les-Moulineaux (FR)
(72) Inventeur: Bellon, Franck, 91160 Longjumeau (FR)
(74) Mandataire: Desaix, Anne

(56) Documents cités:
- EP-A- 0 526 271
- EP-A- 0 666 476
- EP-A- 1 048 949
- WO-A-97/30347
- GB-A- 2 261 508
- US-A- 5 137 614
- US-A- 5 238 651

## Description

L'invention a pour objet un masque pour le dépôt et l'étalement d'un ou plusieurs réactifs sur un support d'analyse, notamment un support d'électrophorèse, par exemple un gel d'agarose.

L'invention est utilisable par exemple dans le domaine de la détection et de la caractérisation de constituants présents dans un échantillon biologique en particulier un liquide biologique tel que sérum, urine, liquide céphalorachidien. En particulier, cette détection peut être réalisée à la suite de la séparation desdits constituants à partir de l'échantillon biologique, par exemple par la technique d'électrophorèse. La détection peut alors être réalisée notamment par les techniques connues d'immunofixation qui requièrent la mise en contact et l'incubation de réactifs avec les constituants séparés de l'échantillon, pour permettre une réaction de reconnaissance immunologique des constituants séparés de l'échantillon biologique avec les réactifs, en des zones déterminées d'un support d'analyse.

L'invention est utilisable avantageusement pour des analyses en routine, pratiquées notamment dans le cadre d'analyses cliniques.

L'invention a également pour objet un masque, destiné au dépôt et à l'étalement d'un ou plusieurs réactifs sur un support d'analyse, associé dans un dispositif à des moyens de positionnement, qui permettent le positionnement du masque par rapport au support d'analyse, à proximité dudit support, lorsque le masque est utilisé pour réaliser le dépôt et l'étalement des réactifs.

Ces moyens de positionnement peuvent aussi être associés à des moyens de guidage, ou peuvent comprendre des moyens de guidage, pour le déplacement du masque lorsqu'il est positionné à proximité du support d'analyse, de façon à permettre l'étalement des réactifs sur des zones délimitées du support, incluant des zones dites d'incubation des réactifs avec les constituants de l'échantillon.

Le dispositif de l'invention peut permettre le dépôt et l'étalement de réactifs en mode manuel. Il peut être aussi aménagé pour permettre le dépôt automatisé de ces réactifs et éventuellement leur étalement automatisé.

L'étape de chargement des réactifs au niveau du masque peut aussi être, selon les modes de réalisation de l'invention, réalisée manuellement ou de façon automatisée. Le masque selon l'invention est avantageusement chargé de façon plus aisée que les masques disponibles dans l'état de la technique.

L'invention a aussi pour objet un procédé de dépôt et d'étalement de réactifs sur un support d'analyse.

Un tel procédé est, selon un mode de réalisation particulier de l'invention, utilisé pour le dépôt et l'étalement de réactifs, destinés à la réalisation d'une immunofixation, en vue de la détection et le cas échéant de la quantification de constituants spécifiques contenus dans un échantillon biologique, lesdits constituants étant préalablement séparés par électrophorèse sur un support tel qu'un gel d'agarose.

L'invention concerne aussi un procédé d'immunofixation, mettant en oeuvre le susdit masque.

L'invention a encore pour objet un kit comprenant un masque selon l'invention.

Un kit selon l'invention est avantageusement adapté pour la mise en oeuvre d'un procédé d'immunofixation au moyen du masque de l'invention.

L'invention concerne aussi les moyens de positionnement et de guidage du masque.

On rappelle que la technique d'immunofixation qui permet notamment l'analyse d'échantillons biologiques en vue du typage des paraprotéines qu'ils contiennent, est une analyse courante largement pratiquée notamment dans les laboratoires d'analyse clinique.

Cette technique, qui associe une électrophorèse, à la formation de précipités sur le gel d'électrophorèse, est connue depuis longtemps. Cette technique a notamment été décrite par Alper CA et Johnson AM Vox. Sang. 17 :445 (1969), Cawley LP et al Clin. Chem. 22 : 1262 (1976), Ritchie RF et Smith R Clin. Chem. 22 :497,1735,1982 (1976). Elle permet l'identification d'anomalies dans différents échantillons biologiques, en particulier dans des liquides biologiques, par exemple dans du sérum, de l'urine ou du liquide céphalo-rachidier.

Cette technique comporte principalement les étapes suivantes :
1) la séparation des constituants protéiques du sérum ou du liquide testé par électrophorèse sur un support tel qu'un gel, par exemple sur gel d'agarose ;
2) la réaction immunologique avec des anticorps spécifiques des protéines séparées ;
3) la révélation des complexes immunologiques formés.

Les conditions pour la réalisation de ces étapes ont été décrites dans l'art antérieur.

La demande de brevet européen EP 1 335 201 A1 décrit par exemple un système formant un contact physique entre un gel et une plaque, ladite plaque comportant des projections venant couper le gel, ou du moins créer une dépression à la surface du gel, afin de former des cavités dont la base est constituée par le gel, les côtés par les projections, et le haut par la partie inférieure de la plaque.

Les dispositifs utilisés comportent en outre la possibilité de réaliser sur le même support d'électrophorèse, notamment le même gel, une piste témoin obtenue par fixation de l'ensemble des protéines séparées présentes dans l'échantillon, à l'aide d'un fixateur de protéines y compris par exemple d'un antisérum polyvalent.

Les nouvelles techniques semi automatiques d'application des échantillons biologiques à analyser, de migration à température contrôlée et de dépôt des réactifs (comprenant par exemple les antisérums et les fixateurs) permettent une miniaturisation des profils d'immunofixation tout en conservant des sensibilités et des résolutions satisfaisantes. Cette miniaturisation autorise l'analyse d'un plus grand nombre d'échantillons sur un même support d'électrophorèse, en particulier sur un même gel.

Ainsi, sur un gel d'électrophorèse de dimensions 8 x 10 cm, on est passé en quelques années de la réalisation d'une à neuf immunofixations (au moyen par exemple du kit d'immunofixation commercialisé sous la marque Hydragel 9IF par la société SEBIA). Cela entraîne des gains de temps au niveau de l'analyse ainsi que des réductions au niveau de la consommation des réactifs qui aboutissent à une diminution du prix de l'analyse.

Pour effectuer le dépôt des réactifs en vue d'une immunofixation dans de telles conditions, le brevet européen EP 0 526 271 B1 décrit par exemple un dispositif ou masque, pour la répartition des réactifs, généralement des Antisérums spécifiques et un Fixateur, capable de résoudre un certain nombre de problèmes posés par les dispositifs ou masques de l'art antérieur et apportant une plus grande sécurité et facilité d'utilisation. Il n'en demeure pas moins que pour réaliser par exemple 9 immunofixations sur un même gel d'électrophorèse en 3 rangées de 3 échantillons au moyen du masque décrit dans le brevet EP 0 526 271 B1, il est nécessaire, pour chaque échantillon, d'effectuer le pipetage de 6 réactifs (Fixateur, Antisérum anti-lgG, Antisérum anti-IgA, Antisérum anti-IgM, Antisérum anti-k et Antisérum anti-λ) soit au total 54 pipetages.

Ces pipetages effectués manuellement peuvent s'avérer longs et fastidieux et ceci malgré l'utilisation possible de pipettes permettant la distribution répétitive.

La présente invention a notamment pour but d'améliorer les conditions de dépôt et d'étalement de réactifs sur un support d'analyse au moyen de masques, en proposant un masque qui permet notamment de réduire le nombre de pipetages de ces réactifs et qui permet de réduire la quantité de réactifs utilisée. Les moyens proposés dans le cadre de l'invention peuvent être utilisés dans toute technique d'analyse requérant le dépôt contrôlé de réactifs sur un support d'analyse. A cet égard, on mentionnera les techniques d'immunofixation suivant une séparation électrophorétique, les techniques d'étalement d'un substrat déterminé, afin de développer une révélation enzymatique, par exemple pour l'analyse de la lactodéhydrogénase (LDH) ou de la créatine kinase (CK).

L'invention a en premier lieu pour objet un masque, convenant pour le dépôt et l'étalement de réactifs sur un support d'analyse, dont la conception tient compte d'une utilisation incluant son déplacement, pour la réalisation de l'étape d'étalement des réactifs sur les zones déterminées du support d'analyse. Le masque de l'invention peut de ce fait être présenté comme étant un masque mobile en situation d'utilisation. La présente invention a également pour effet de limiter la consommation des réactifs, en particulier des antisérums qui sont des produits coûteux et du fixateur dans la cas des réactions d'immunofixation, et donc de diminuer le coût des analyses effectuées. Elle a aussi pour effet de faciliter le chargement des réactifs dans le masque, en particulier en limitant le nombre de pipetages et/ou en autorisant le chargement automatisé du masque.

De plus, le masque proposé assure une qualité de résultat constante, dans des conditions de manipulation améliorées, voire simplifiées. En particulier, il devient inutile après la phase d'incubation des réactifs étalés au moyen du dispositif de l'invention d'éliminer les excès de réactifs qui restent entre le gel et le masque comme cela est nécessaire lors de l'utilisation d'un masque tel que proposé dans le brevet EP 0 526 271 B1.

En effet, avec le masque selon l'invention, en fin de répartition et d'étalement des réactifs, il n'y a plus de réactif libre entre le masque et le support d'analyse, la totalité des réactifs initialement introduits ayant été déposée sur le support d'analyse. Il n'est donc pas nécessaire de pomper un excès de réactifs qui serait présent sur le support d'analyse.

L'invention a donc pour objet un masque, convenant pour le dépôt et l'étalement de réactifs sur un support d'analyse d'échantillons biologiques, comprenant :
- une surface inférieure et une surface supérieure au moins en partie parallèles entre elles, séparées par une distance constituant l'épaisseur du masque,
- une ou plusieurs piste(s), correspondant à des zone(s) délimitée(s), localisées au niveau de la surface inférieure du masque et comprenant un élément en saillie faisant saillie à la surface inférieure du masque, chaque élément en saillie comprenant une partie constituant une pente par rapport à un plan horizontal,
- associée à chaque piste, une ouverture pour le chargement et le dépôt de réactifs, ladite ouverture traversant le masque sur la totalité de son épaisseur depuis un orifice supérieur de chargement à la surface supérieure du masque jusqu'à un orifice inférieur de dépôt, ledit orifice inférieur de dépôt étant situé dans la penteiste, à proximité du point le plus bas de la pente de la piste,
ce masque étant tel que la ou les pistes qu'il comporte permettent le maintien par capillarité entre la piste et la surface du support d'analyse en regard de laquelle serait placé le masque, de réactifs chargés au niveau de chaque ouverture et déposés sur le support d'analyse.

L'expression "masque" désigne de façon générale, dans la présente invention une plaque conçue de façon à permettre, le cas échéant en collaboration avec des moyens associés, le positionnement en regard de zones repérées d'un support d'analyse, où doit se faire le dépôt et l'étalement de réactifs lorsqu'ils ont été chargés dans le masque et amenés au contact des zones repérées.

Les dimensions du masque de l'invention sont de préférence telles qu'il ne couvre pas la totalité de la surface du support d'analyse sur laquelle les réactifs chargés dans ce masque doivent être déposés et étalés, lorsque le masque est positionné à proximité du support d'analyse pour être utilisé. La largeur du masque (dans laquelle s'inscrit la longueur des pistes) est en particulier inférieure à la longueur des pistes de migration électrophorétiques du support d'analyse, puisque la longueur des pistes du masque est inférieure à la longueur des pistes de migration électrophorétiques du support d'analyse ; l'étalement des réactifs sur ces pistes résulte en conséquence du déplacement du masque au dessus du support d'analyse comme décrit ci-après conjointement à l'avancée des réactifs sur ledit support permise par de la structure des pistes du masque.

Le masque de l'invention est donc destiné à être déplacé au dessus du support d'analyse, pour permettre l'étalement des réactifs.

L'ouverture est dite "associée à chaque piste", ce qui signifie dans le cadre de la présente demande, que son orifice inférieur est localisé de façon à apporter le réactif chargé dans l'ouverture du masque, au niveau de la pente de la piste, de façon à permettre le dépôt du réactif sur le support d'analyse et son maintien par capillarité entre le support et la piste, ainsi que son étalement sur le support d'analyse, lors du déplacement du masque.

Ladite ouverture est par exemple un trou perpendiculaire à la surface supérieure du masque, traversant le masque de part en part. L'orifice inférieur de l'ouverture est situé dans la pente, à proximité du point le plus bas de la pente. En étant localisé « à proximité » du point le plus bas - par rapport à un plan horizontal - de la pente de la piste, l'orifice inférieur de l'ouverture permet un étalement du réactif liquide au niveau de la piste, par une remontée du réactif le long de la piste.

L'orifice supérieur de l'ouverture peut être à la verticale de l'orifice inférieur. Alternativement, il peut être positionné suivant un plan incliné par rapport à cette verticale dès lors qu'il permet l'apport du réactif au niveau de l'orifice inférieur dans des conditions compatibles avec le dépôt et l'étalement de ce réactif sur le support d'analyse.

Avantageusement, une ouverture associée à une piste, constituée par un trou perpendiculaire à la surface supérieure du masque et le traversant de part en part, est formée par un orifice circulaire, débouchant au niveau de la surface supérieure du masque, qui se prolonge par une partie tronconique, se terminant par exemple par une partie cylindrique débouchant au niveau de la surface inférieure du masque, par un orifice situé dans la pente de la piste, à proximité du point le plus bas de la pente. La présence d'une partie cylindrique débouchant au niveau de l'orifice inférieur permet de répartir la pression que peut exercer par exemple une pipette sur les bords de l'orifice inférieur lors du chargement des réactifs, d'où une amélioration de la résistance du masque.

L'ouverture tronconique permet en outre avantageusement de guider une pipette de remplissage et permet d'assurer l'étanchéité entre l'extrémité de la pipette et le masque lors de l'injection du réactif entre la piste et le support d'analyse.

Le cas échéant, l'ouverture est modifiée par rapport à la précédente, en ce que la partie conique se prolonge jusqu'à l'orifice supérieur de l'ouverture, par une partie cylindrique à section circulaire.

Selon un mode particulier de l'invention, l'ouverture ainsi décrite peut aussi être telle que l'orifice débouchant au niveau de la surface supérieure du masque est plus grand que l'orifice inférieur de l'ouverture, par exemple le diamètre du premier est supérieur à celui du second si ces orifices sont circulaires.

Un masque particulier selon les définitions précédentes de l'invention, convenant pour l'étalement de réactifs sur un support d'analyse d'échantillons biologiques, peut être défini comme comprenant :
- une surface inférieure et une surface supérieure au moins en partie parallèles entre elles, séparées par une distance constituant l'épaisseur du masque,
- une ou plusieurs pistes comprenant chacune un élément en saillie de forme allongée, émergeant sous la surface inférieure du masque, ledit élément en saillie comprenant une partie constituant une pente par rapport à un plan horizontal,
- associée à chaque piste, une ouverture traversant le masque sur la totalité de son épaisseur depuis un orifice supérieur présent à la surface supérieure du masque jusqu'à un orifice inférieur, ledit orifice inférieur étant situé dans la piste, à proximité du point le plus bas de la pente de la piste,
ce masque étant tel que la ou les pistes qu'il comporte permettent le maintien par capillarité entre la piste et la surface du support d'analyse en regard de laquelle serait placé le masque, de réactifs chargés au niveau de l'ouverture et déposés sur le support d'analyse.

Les pistes du masque ayant une forme allongée peuvent aussi être appelées « rampes ». Leurs pentes sont toutes inclinées dans le même sens. Lesdites pentes sont destinées à retenir le réactif par capillarité comme indiqué ci-dessus et à assurer le rassemblement du réactif au point le plus bas de la pente de façon à, lors du déplacement du masque, permettre son étalement.

La demande décrit encore un autre masque entrant dans le cadre des définitions qui précèdent, comprenant:
- une surface inférieure et une surface supérieure au moins en partie parallèles entre elles, séparées par une distance constituant l'épaisseur du masque,
- une ou plusieurs pistes comprenant chacune un élément en saillie émergeant sous la surface inférieure du masque, constitué par une protubérance de forme parallélépipédique tronquée, ledit élément en saillie comprenant une partie constituant une pente par rapport à un plan horizontal,
- associée à chaque piste, une ouverture traversant le masque sur la totalité de son épaisseur depuis un orifice supérieur présent à la surface supérieure du masque jusqu'à un orifice inférieur, ledit orifice inférieur étant situé dans la piste, à proximité du point le plus bas de la pente de la piste,
ce masque étant tel que la ou les pistes qu'il comporte permettent le maintien par capillarité entre la piste et la surface du support d'analyse en regard de laquelle serait placé le masque, de réactifs chargés au niveau de l'ouverture et déposés sur le support d'analyse.

Dans le cas où l'élément en saillie est constitué par un élément de forme allongée ou est constitué par une protubérance de forme parallélépipédique tronquée, ledit élément en saillie a une surface supérieure coïncidant avec la partie de la surface inférieure du masque dont il émerge, et une surface inférieure séparée de la surface supérieure selon au moins une pente par rapport à un plan horizontal, le point le plus bas de ladite pente situé à proximité dudit orifice inférieur de l'ouvérture de chaque piste, étant le point le plus proche du support d'analyse en regard et à proximité duquel il est amené en position d'utilisation.

L'élément en saillie comprenant une pente émergeant à la face inférieure du masque, permet avantageusement le dépôt et l'étalement de réactifs chargés dans le masque, par déplacement du masque le long d'une zone du support d'analyse venant en regard de la ou des pistes du masque, dans un plan horizontal par rapport à ce support.

Selon un exemple de la demande, le masque est pourvu d'une ou plusieurs pistes comprenant chacune un élément en saillie émergeant sous sa surface inférieure, ledit élément en saillie incluant une sphère creuse dont la cavité est destinée à recevoir un réactif, et dont les dimensions sont adaptées à la largeur de la zone du support d'analyse devant être couverte par le réactif, ledit réactif étant maintenu par capillarité dans la cavité de la sphère et distribué sur le support d'analyse par un trou réalisé dans la sphère, lors du déplacement du masque. Un tel masque répond par ailleurs aux caractéristiques énoncées pour les éléments en saillie de forme allongée.

Selon un mode de réalisation particulier de l'invention, les surfaces inférieure et supérieure du masque sont totalement parallèles entre elles, en dehors des zones constituant les pistes, voire en dehors des zones constituant les pentes des pistes.

Dans le cadre des modes de réalisation de l'invention décrits ci-dessus, la pente présente au niveau de chaque piste du masque, quelle que soit la forme de l'élément en saillie, coïncide avec la piste.

Alternativement, la pente peut s'étendre sur une partie seulement de la piste. Par exemple la piste comportant l'orifice inférieur au point le plus bas de sa pente peut se prolonger au delà de ladite pente, par exemple dans un plan horizontal. Selon une autre variante, la pente peut être constituée de plusieurs pentes.

Lorsqu'un masque selon l'invention est utilisé en association avec un support d'analyse d'échantillons biologiques, par exemple un gel d'électrophorèse, ce masque est amené « à proximité » du support d'analyse: ceci signifie que le masque n'entre pas en contact avec les zones du support sur lesquelles doivent se faire le dépôt et l'étalement du ou des réactifs (encore appelées zones d'incubation des réactifs) et qu'en outre le ou les réactifs déposés sur ledit support sont maintenus, par l'effet des forces de capillarité, entre les pistes du masque et ledit support d'analyse, ce qui permet leur étalement sur des zones déterminées de ce support d'analyse situées en regard des pistes du masque lors de son déplacement parallèlement au support d'analyse, au dessus de ce support. L'absence de contact ci-dessus définie s'entend plus précisément, dans certains modes de réalisation de l'invention, d'une absence de contact qui suit l'instant de l'initiation de la descente des réactifs pour les déposer sur le support d'analyse, cette descente pouvant en effet, selon un mode de réalisation particulier de l'invention, faire intervenir un contact passager avec le support d'analyse comme illustré ci-après.

La pente formée au niveau de l'élément en saillie de chaque piste du masque est telle que son point dit le plus bas est le point le plus proche du plan horizontal constitué par le support d'analyse en position d'utilisation. En conséquence, le point dit le plus haut de la pente de ladite piste est le point le plus éloigné du plan horizontal constitué par le support d'analyse dans cette situation de fonctionnement. La face inférieure de chaque piste du masque en regard du support est donc inclinée par rapport à l'horizontale.

La position des pistes par rapport au plan horizontal du support d'analyse, et la position de l'orifice inférieur de l'ouverture associée à chaque piste permettent lors de l'étalement des réactifs déposés sur le support d'analyse, d'en d'assurer la venue au point le plus bas de la pente de la piste, puisqu'en ce point, les forces de capillarité exercées sur les réactifs sont les plus grandes.

Le liquide constituant le réactif peut être réparti sur la totalité de la piste ou sur une partie seulement de la piste.

Ainsi, le dépôt et l'étalement du ou des réactifs au moyen du masque selon l'invention peuvent se faire de façon contrôlée sur des zones délimitées du support d'analyse, sans qu'un contact soit établi entre les pistes du masque et le support d'analyse.

En dehors des zones du support d'analyse venant en regard des pistes du masque lors de son utilisation, un contact peut être établi entre le masque et le support d'analyse, dès lors qu'en mouvement, le masque peut glisser parallèlement au support d'analyse, le long de ce support, sans l'endommager.

Selon un mode de réalisation particulier de l'invention, aucun point du masque en situation d'utilisation ne vient au contact du support d'analyse, à l'exception éventuellement de zones du masque permettant son positionnement à proximité du support d'analyse.

Il est préférable de prévoir que l'appui du masque nécessaire à son positionnement à proximité du support d'analyse, soit réalisé à l'extérieur dudit support d'analyse, par exemple sur le plan (ou plateau) sur lequel le support est déposé.

Lorsqu'il est question de la surface supérieure et de la surface inférieure du masque, des pistes ou des pentes, ces notions sont entendues par référence à la position du masque au dessus d'un support d'analyse lui-même en position horizontale. En d'autres termes, la surface inférieure du masque et la surface inférieure de chaque piste, ou de chaque pente, sont en regard de la surface du support d'analyse lorsque le masque est en position d'utilisation. Le plan horizontal par rapport auquel est définie la pente de l'élément en saillie, peut donc être celui du support d'analyse lorsqu'il est utilisé dans une position horizontale.

L'invention a également pour objet un masque dont l'utilisation pour déposer et étaler des réactifs sur un support d'analyse, fait appel au principe mis en oeuvre dans le cadre de l'invention, du maintien par capillarité des réactifs entre une pente définie au niveau du masque et une zone du support d'analyse en regard de ladite piste, ledit masque se distinguant du masque défini précédemment par le fait que la pente constituée au niveau de chaque piste du masque, est réalisée par l'inclinaison conférée au masque par rapport au support d'analyse, lors de son utilisation. La surface inférieure du masque est dans ce cas parallèle à la surface inférieure de l'élément en saillie qui vient en regard du support d'analyse, la pente résultant du positionnement incliné du masque par rapport au support d'analyse.

Dans le cadre de ce mode de réalisation particulier, les caractéristiques des pistes définies pour les masques dont la pente est intégrée à la piste sont transposables lorsque la pente est constituée du fait de l'utilisation du masque en position inclinée par rapport au support d'analyse.

Ainsi, lorsque la piste est de forme allongée, elle peut être de forme parallélépipédique, délimitant par sa forme la zone de maintien des réactifs par capillarité entre la piste et le support d'analyse. En situation d'utilisation, l'ouverture traversant le masque est située en un point de la piste qui se trouve à proximité du point le plus bas de la pente formée par l'inclinaison relative du masque par rapport au support d'analyse.

Le masque selon l'invention est de dimensions compatibles avec les dimensions du support d'analyse sur lequel on veut déposer et étaler des réactifs et les pistes de ce masque sont de formes et de dimensions compatibles avec le volume de réactif liquide que l'on souhaite déposer et étaler sur le support d'analyse et avec la forme et à la dimension des zones délimitées du support d'analyse sur lesquelles doivent être réalisés le dépôt et l'étalement des susdits réactifs, par exemple en vue de leur incubation avec les constituants de l'échantillon biologique.

Pour déposer et étaler un ou plusieurs réactifs sur un support d'analyse d'échantillons biologiques lorsque le masque et le support d'analyse sont disposés parallèlement l'un par rapport à l'autre, on déplace le masque dans un plan horizontal au dessus du plan du support d'analyse, à partir de la zone du support d'analyse au niveau de laquelle est initialement positionné le masque et qui correspond au point de dépôt initial des réactifs, de façon à étaler les réactifs sur les zones du support d'analyse venant en regard des pistes du masque.

Pour déposer et étaler un ou plusieurs réactifs sur un support d'analyse d'échantillons, lorsque la pente des pistes du masques résulte non pas de la structure des pistes, mais de l'inclinaison conférée au masque par rapport au support d'analyse (ou inversement), on déplace le masque dans un plan incliné donné, selon des modalités identiques à celles décrites pour le masque dont les pistes comportent une pente dans leur structure.

Chaque déplacement permettant un passage du masque au dessus de la totalité des zones repérées du support d'analyse devant recevoir les réactifs, est appelé « balayage ». Un premier balayage peut par exemple être fait de la zone correspondant à l'anode d'un support d'électrophorèse vers la zone correspondant à la cathode d'un tel support, ou dans le sens inverse de la cathode vers l'anode, de façon à couvrir la totalité des zones délimitées devant recevoir les réactifs, ces zones correspondant dans le cas d'un support (tel qu'un gel) d'électrophorèse, aux pistes de migration électrophorétique. Dans la suite, conformément à ce qui est indiqué ci-dessus, lorsqu'il est question de l'anode pour situer la localisation d'un dépôt de réactif ou le sens du mouvement du masque par rapport au support d'analyse, il est entendu que cette localisation ou ce mouvement peut, à l'inverse, être effectué à partir de la cathode.

Le masque conçu selon l'invention permet donc, lors de son déplacement par balayage de la surface déterminée du support d'analyse après le chargement des réactifs au niveau de l'ouverture associée à chaque piste, de réaliser le dépôt et l'étalement desdits réactifs sur la totalité des zones déterminées du support d'analyse, pour l'ensemble des échantillons biologiques testés se succédant dans la direction du déplacement du masque.

Il devient donc inutile de multiplier le pipetage de chaque réactif pour chaque échantillon à traiter. Le nombre de pipetages à effectuer correspond au nombre de réactifs devant être déposés sur une rangée du support d'analyse, et correspond donc normalement au nombre d'ouvertures prévues dans le masque, lorsque toutes les pistes du masque sont utilisées.

En outre, la quantité de chaque réactif chargée au niveau du masque peut être considérablement diminuée par rapport à la quantité de chaque réactif normalement utilisée lorsque chaque réactif doit être chargé pour chacun des échantillons présents sur le support d'analyse.

A titre d'exemple, si le masque selon l'invention est destiné au dépôt et à l'étalement de réactifs pour réaliser une immunofixation, en vue de détecter des constituants particuliers d'un échantillon biologique préalablement analysé par électrophorèse, on observe que pour l'utilisation de 6 réactifs pour chaque échantillon testé (soit généralement un fixateur capable de fixer les constituants de l'échantillon de façon à réaliser un profil témoin au niveau du support d'électrophorèse, et des antisérums spécifiques anti- IgG, IgA, IgM, κ et λ), la quantité de chaque réactif chargé sur le masque est diminuée de 4,5 fois par rapport à la quantité chargée, pour chaque échantillon à traiter, lors de l'utilisation d'un masque fixe tel que celui du brevet EP 0 526 271.

La quantité de chaque réactif chargée au niveau de chaque piste du masque est déterminée en fonction de la dimension de la zone d'incubation à recouvrir au moyen de ce réactif, par exemple en fonction du nombre de rangées d'échantillons se succédant dans la direction de déplacement du masque. Lorsque le masque est utilisé pour le dépôt et l'étalement de réactifs en vue d'une immunofixation, la zone d'incubation comprend ou coïncide avec la zone du support d'électrophorèse qui comprend le profil électrophorétique des échantillons à analyser.

A titre d'exemple, au moyen d'un masque comportant trois groupes de six pistes sur une rangée, on peut réaliser une immunofixation de neuf échantillons, voire de 12 ou 18 échantillons (répartis par exemple en rangées de 3 échantillons différents, chaque échantillon occupant 6 pistes du support d'analyse pour l'électrophorèse et donc du masque.

Le masque est avantageusement chargé avec une quantité de réactifs telle que chaque zone déterminée du support d'analyse sur laquelle doit être étalé un réactif, est déjà couverte de façon homogène par ce réactif, en un balayage. Pour déterminer le volume chargé de chaque réactif, ont tient compte de la course du masque par balayage du support d'analyse et de la largeur de la zone à recouvrir sur le support d'analyse. De façon générale, on retient que la quantité de chaque réactif chargé varie entre 4µl et 15µl, et par exemple est une quantité de 15, 10, 8, 6 ou 4µl pour chaque réactif.

A titre d'exemple, on observe qu'un volume de 4µl de réactif peut suffire à couvrir une surface de support d'analyse de 175 mm², de façon homogène, correspondant à un étalement de réactif de 0;02µl/mm² de support d'analyse.

De préférence, le masque selon l'invention est un masque rigide, ou rigidifié notamment par association avec des moyens de rigidification qui peuvent par exemple, participer au positionnement et/ou au guidage du masque.

Le choix du matériau utilisé pour réaliser le masque n'est pas limité en principe.

Le masque peut être par exemple réalisé en un matériau susceptible d'être moulé, de fournir une surface lisse, notamment en matière plastique.

Le matériau peut être transparent ou translucide et on citera à titre d'exemples les matériaux tels que polycarbonate, polymétacrylate polyéthylène, polystyrène cristal, plexiglas.

Le masque selon l'invention peut être de type jetable après une utilisation.

La rigidité conférée au masque permet de s'assurer de son déplacement dans un plan déterminé, horizontal ou incliné, par rapport au support d'analyse, lorsqu'il est associé, par exemple encliqueté, aux moyens de positionnement et aux moyens de guidage appropriés.

Dans un mode de réalisation préféré de l'invention, un masque répondant aux caractéristiques définies dans les pages précédentes comporte plusieurs pistes parallèles entre elles, réparties sur la longueur du masque.

Lorsque, dans le cas le plus fréquent, le masque comporte plusieurs pistes parallèles entre elles, l'écartement entre les pistes (écartement interpistes) est déterminé en fonction du nombre de pistes sur le masque, de la nécessité de prévenir d'éventuelles interactions entre les réactifs, en particulier entre le fixateur et les antisérums.

Avantageusement, l'écartement entre les différentes pistes parallèles est constant. Cet écartement peut être faible, par exemple inférieur à 3mm, et en particulier de l'ordre de 2,5mm, étant de préférence supérieur ou égal à 2mm, en particulier pour empêcher une interaction éventuelle entre le fixateur et l'antisérum de la piste voisine.

La zone du support d'analyse au niveau de laquelle a lieu l'étalement de chacun des réactifs a une largeur au moins égale à la largeur de la piste du masque mise en regard de cette zone.

A titre d'exemple, la largeur de la piste et la largeur de la surface d'incubation, qui peut correspondre à l'étalement du profil électrophorétique sur le support d'analyse, sont semblables et d'environ 2,5 mm. Selon un autre exemple la largeur en question est de 3,5mm.

Pour éviter que se produise une interaction entre réactifs voisins, on peut en outre disposer les pistes du masque de façon à prévenir le chevauchement des différentes zones de dépôt des réactifs ou des zones de diffusion éventuelle des réactifs déposés sur le support d'analyse ou réaliser le chargement des réactifs et leur descente sur le support d'analyse, de façon à prévenir une éventuelle interaction. Des modes de chargement et de descente des réactifs sont décrits ci-après, incluant par exemple mais pas nécessairement, un chargement en dehors du support d'analyse, qui permettent d'éviter ce type d'interaction tout en maintenant la totalité des pistes parallèles, dans un seul alignement.

S'agissant des interactions discutées ci-dessus, il convient en particulier de s'assurer que le réactif destiné à la réalisation du profil témoin de chaque échantillon n'interagit pas avec les réactifs spécifiques (notamment les antisérums), ce qui pourrait fausser la détection de constituants particuliers de l'échantillon.

Pour ce faire, lorsque des moyens spécifiques sont prévus dans la structure du masque, un premier mode de réalisation du masque comprenant plusieurs pistes parallèles entre elles, réparties sur la longueur du masque avec un écartement constant, consiste à prévoir une première série de pistes parallèles entre elles, réparties sur la longueur du masque et une deuxième série de pistes parallèles entre elles et parallèles à la première série des pistes et dont les orifices inférieurs sont situés dans le même plan horizontal, cette deuxième série de pistes formant un alignement décalé par rapport à l'alignement formé par la première série de pistes.

Selon une variante de réalisation du masque, l'alignement décalé de la deuxième série de pistes est remplacé par l'augmentation de l'écartement entre lesdites pistes de la susdite première série et les autres pistes de la susdite deuxième série.

Le décalage de la deuxième série de pistes ou l'écartement de cette série de pistes par rapport aux pistes de la première série, est destiné à prévenir l'interaction entre des réactifs de la deuxième série de pistes et ceux des autres pistes, lors de leur dépôt sur le support d'analyse. En général ces pistes décalées ou plus écartées sont destinées à recevoir le fixateur dans une immunofixation, capable de fixer les protéines du profil électrophorétique de façon à réaliser un profil témoin.

Lorsque le masque ne comporte pas une série de pistes ainsi décalées ou ayant un écartement différencié par rapport à celui des autres pistes, l'interaction entre les réactifs en question peut être évitée, si besoin, en effectuant par exemple le chargement et le dépôt de ces réactifs séparément, en deux temps, au niveau du support d'analyse.

Par exemple, on réalise le dépôt du fixateur du côté anodique du support d'électrophorèse, puis on l'étale par balayage avant de charger les antisérums, par exemple en position anodique mais décalée, par exemple d'environ 5mm vers la cathode par rapport à la position du premier chargement. Alternativement le chargement des antisérums est réalisé en position cathodique.

Le recours au chargement en deux temps n'est toutefois pas toujours nécessaire, en particulier si les modalités de chargement des réactifs ne conduisent pas à une interaction gênante entre les réactifs. Une telle interaction peut par exemple être évitée même si tous les réactifs sont chargés ensemble, par exemple en dehors de la surface d'analyse et lorsque la descente simultanée de tous les réactifs chargés peut être réalisée sur le support d'analyse sans occasionner l'interaction gênante.

Lorsque le masque est réalisé de façon à ce que la pente des pistes résulte de l'inclinaison du masque par rapport au support d'analyse et non pas de la structure des pistes, le décalage des pistes ou groupes de pistes n'est pas nécessaire, les réactifs devant alors être chargés et déposés en plusieurs temps selon leur nature.

Le masque selon l'invention peut être réalisé de façon à ce que les pistes soient organisées en plusieurs groupes, chaque groupe étant par exemple constitué par une piste positionnée de façon décalée par rapport aux autres pistes alignées les unes par rapport aux autres, et par les pistes alignées précédant la piste décalée suivante.

Par ailleurs, le masque de l'invention est tel qu'il permet par sa structure et le cas échéant par les conditions de son utilisation, le dépôt et l'étalement de réactifs sans interaction entre les différents réactifs déposés sur les différentes zones du support d'analyse.

On a pu observer, notamment au niveau de la zone de dépôt initiale des réactifs sur le support d'analyse, préalablement à leur étalement, un phénomène localisé de diffusion desdits réactifs sur le support d'analyse.

Pour éviter les conséquences éventuelles des phénomènes de diffusion localisée des réactifs au moment de leur dépôt, on peut avantageusement choisir de déposer les réactifs au moyen du masque, en dehors de la zone du support d'analyse susceptible de comporter les constituants des échantillons à détecter, donc par exemple en dehors de la zone comportant les profils électrophorétiques.

Dans le cas d'une immunofixation, on dépose par exemple lesdits réactifs dans une zone située en dehors de celle correspondant au profil électrophorétique, par exemple du côté anodique du support par rapport à ces profils.

Avantageusement, le masque de l'invention est tel que l'ouverture le traversant de part en part est perpendiculaire aux surfaces supérieures et inférieures du masque.

La forme des ouvertures du masque doit permettre d'y charger une quantité de réactifs suffisante pour permettre le dépôt et l'étalement de ce réactif sur la totalité de la zone déterminée du support d'analyse sans devoir procéder à un nouveau chargement du masque avec ledit réactif.

En outre, cette ouverture à une forme et une localisation compatibles au dépôt et au maintien par capillarité d'une quantité donnée de réactif entre la piste du masque et le support d'analyse, jusqu'à épuisement de la quantité introduite au cours de l'opération d'étalement du réactif sur le support d'analyse.

Lors du dépôt des réactifs sur le support d'analyse, il convient, quand le chargement du masque est effectué au-dessus du support d'analyse avec remplissage des pistes au fur et à mesure du chargement des réactifs, d'assurer l'étanchéité entre le masque et l'extrémité de la pipette ou de tout autre moyen utilisé pour charger les réactifs dans le masque.

Le volume de l'ouverture peut par ailleurs être prévu pour permettre le chargement des réactifs à l'extérieur du support d'analyse, les réactifs devant dans ce cas être retenus dans leur totalité par capillarité dans l'ouverture, jusqu'à leur dépôt sur le support d'analyse. Dans ce cas, le masque est ensuite positionné de façon à assurer le dépôt des réactifs qu'il contient, sur le support d'analyse. Ce positionnement doit donc permettre d'établir le contact, éventuellement par des modalités particulières, entre le liquide et le support d'analyse.

Avantageusement, l'ouverture présente au niveau de chaque piste peut recevoir le réactif en excès par rapport à la quantité de réactif nécessaire à la réaction.

Le masque selon l'invention permet d'utiliser des quantités réduites de réactifs, par exemple d'environ 15µl, ou 10 µl. Cependant, les dimensions de l'ouverture traversant le masque et destinée à recevoir ces réactifs, peuvent être déterminées pour accepter une quantité de réactifs supérieure à la quantité effectivement mise en oeuvre. Par exemple, le volume de l'ouverture peut permettre d'accepter une quantité de réactif allant jusqu'à environ 30µl.

Les caractéristiques géométriques du masque, en particulier le nombre de pistes, l'écartement entre pistes (écartement interpistes) sont adaptées au nombre de dépôts, à leur largeur et à l'écartement de ces dépôts effectués par rangées de dépôts sur le support d'analyse. Le masque selon l'invention a ainsi une longueur suffisante pour accueillir plusieurs pistes selon un alignement, et une largeur limitée (plus petite) par rapport à la longueur de la piste de migration électrophorétique de la surface d'incubation du support d'analyse venant en regard de cette piste lorsque le masque est déplacé pour fonctionner.

Il est entendu et c'est une caractéristique du présent dispositif, que le même masque peut être utilisé pour révéler sur un même gel plusieurs rangées de dépôts ayant les mêmes caractéristiques en nombre et en dimensions. Ces rangées de dépôts ont été réalisées sur le support d'analyse, en rangées parallèles entre elles, et perpendiculaires à la direction de la migration électrophorétique.

Avantageusement, la géométrie du masque selon l'invention permet le dépôt et le maintien du réactif par capillarité entre chaque piste du masque et le support d'analyse, lorsque la distance entre le masque et le support d'analyse est inférieure ou égale à 2 mm de préférence comprise entre 0,1 et 1,5 mm. Cette distance entre le masque et le support d'analyse varie selon le point considéré du masque, en particulier cette distance est de préférence d'environ 0,1 à 0,5 mm au point du masque le plus proche du support (correspondant au point le plus bas de la pente de la piste ou de la pente du masque) et cette distance est de préférence inférieure à 2 mm, avantageusement inférieure ou égale à 1,5 mm, au point du masque le plus éloigné du support d'analyse (correspondant au point le plus haut de la pente de la piste ou de la pente du masque).

Dans ces limites, l'inclinaison de la pente doit être telle que l'écartement du masque par rapport au support d'analyse est compatible avec les forces capillaires qui maintiennent le réactif entre la piste et le support d'analyse.

A titre d'exemple, un masque selon l'invention est réalisé de façon telle que les pistes sont séparées les unes des autres par un écartement supérieur ou égal à 1,5 mm. De préférence, l'écartement entre les pistes est de 2,5 mm. La largeur des pistes est avantageusement de 2,5 mm.

La piste du masque réservée au fixateur peut en outre être décalée, par exemple peut se trouver en position plus anodique que les autres pistes, lorsque le masque est en position d'utilisation, à proximité d'un support d'analyse constitué par un gel d'électrophorèse.

Ainsi un masque particulier est caractérisé en ce que la piste destinée au fixateur n'est pas alignée avec les autres pistes, se trouvant décalée par rapport à l'alignement formé par les autres d'une distance de 5, de préférence 6 à 7 mm.

Un masque particulier convenant à la réalisation de l'invention et en particulier un masque adapté à une utilisation avec un gel d'électrophorèse de longueur 10 cm et de largeur (entre les pôles anodique et cathodique) d'environ 8cm est tel que chaque piste du masque a les dimensions suivantes :
- longueur: 3 à 15 mm
- largeur : 1 à 10 mm
- inclinaison de la pente : 1 à 10° par rapport à l'horizontale.

Un masque particulièrement préféré convenant notamment pour une utilisation avec le gel ci-dessus, est un masque dans lequel chaque piste a les dimensions suivantes :
- longueur : 7 mm
- largeur : 2,5 mm
- inclinaison de la pente : 5° par rapport à l'horizontale.

Dans ces modes de réalisation particuliers, les autres caractéristiques du masque énoncées ci-dessus peuvent naturellement être associées aux caractéristiques particulières ci-dessus. En particulier, l'écartement interpistes est avantageusement de 2,5 mm et/ou le décalage entre l'alignement des pistes des réactifs spécifiques et l'alignement des pistes du fixateur est de 6 à 7 mm.

Encore suivant un mode de réalisation particulièrement avantageux de l'invention, le masque est tel que l'ouverture qui le traverse répondant aux caractéristiques décrites ci-dessus à ce sujet, a une partie conique formant un angle d'environ 50°.

Dans le cadre des différents modes de réalisation de l'invention, l'épaisseur du masque est avantageusement comprise entre 1 et 10 mm.

Dans un mode de réalisation particulier de l'invention, le masque a avantageusement une largeur inférieure à 30mm, de préférence inférieure à 20mm ou à 15mm, de façon encore préférée inférieure à 10mm.

Lorsque le masque selon l'invention est utilisé pour le dépôt et l'étalement de réactifs sur un support d'électrophorèse, il peut encore être caractérisé en ce qu'il est compatible avec les caractéristiques de localisation des échantillons biologiques séparés sur le support d'électrophorèse ; en particulier ledit masque permet :
- l'alignement des rangées des pistes du masque, perpendiculairement à la direction de migration électrophorétique ;
- le positionnement du masque à proximité du support d'analyse, de façon à permettre le maintien des réactifs par capillarité entre les pistes du masque et le support d'analyse ;
- le positionnement transversal du masque par rapport à la direction de migration électrophorétique, pour permettre l'alignement des rangées des migrations électrophorétiques réalisées sur le support d'analyse et des rangées de pistes du masque.

Pour être utilisé avec différents supports d'analyse, et à titre d'exemple, un masque selon l'invention peut comprendre entre 1 et 24 pistes, de préférence entre 6 et 24 pistes, notamment peut comprendre 6, 9, 12, 15 ou 18 pistes.

Un masque comprenant 18 pistes permet, s'il est destiné à l'utilisation dans une réaction d'immunofixation faisant suite à une séparation électrophorétique, de déposer pour trois échantillons différents occupant une même rangée du support d'électrophorèse, et pour un nombre donné de rangées d'échantillons (par exemple 2 ou plus, notamment 3 ou 4), un fixateur pour réaliser un profil témoin et 5 réactifs spécifiques tels que des antisérums, notamment des antisérums anti-IgG, anti-IgA, anti-IgM, anti-κ et anti-λ, pour chaque échantillon. On peut aussi réaliser selon les mêmes modalités un masque à 6 ou 12 pistes.

L'invention a également pour objet un masque tel que défini ci-dessus, associé à des moyens de positionnement destinés à maintenir la surface inférieure des pistes du masque, à proximité de la surface du support d'analyse au voisinage duquel le masque serait amené pour le dépôt et l'étalement de réactifs sur le support d'analyse.

Des moyens de positionnement appropriés peuvent être constitués par des butées susceptibles de reposer sur le support d'analyse en dehors de la surface d'incubation comportant les échantillons biologiques ces butées étant de dimensions telles que le masque ne vient pas au contact du support d'analyse dans sa partie correspondant à la surface d'incubation des réactifs.

Les moyens de positionnement peuvent en outre être associés à des moyens de guidage du masque de façon à permettre son déplacement contrôlé au-dessus du support d'analyse, conformément à ce qui a été énoncé ci-dessus.

L'invention a donc aussi pour objet, un dispositif pour le dépôt et l'étalement d'un ou plusieurs réactifs sur un support d'analyse d'échantillons biologiques comprenant :
a) un masque tel que ci-dessus défini,
b) des moyens de positionnement et de guidage du masque permettant le positionnement du masque de façon telle que le masque est maintenu à proximité de la surface du support d'analyse et permettant le guidage du masque par balayage de la surface du support d'analyse, dans un plan horizontal parallèle à la surface dudit support, pour permettre le dépôt et l'étalement des réactifs sur chacune des zones déterminées du support d'analyse venant en regard des pistes du masque.

Selon une variante de l'invention, le masque mis en oeuvre est tel que la pente des pistes résulte de l'inclinaison du masque par rapport au support d'analyse. Dans ce cas, l'invention vise un dispositif pour le dépôt et l'étalement d'un ou plusieurs réactifs sur un support d'analyse d'échantillons biologiques comprenant :
a) un masque tel que ci-dessus défini,
b) des moyens de positionnement et de guidage du masque permettant le positionnement du masque de façon telle que le masque est maintenu à proximité de la surface du support d'analyse et permettant le guidage du masque par balayage de la surface du support d'analyse, dans un plan incliné déterminé à la surface dudit support, pour permettre le dépôt et l'étalement des réactifs sur chacune des zones déterminées du support d'analyse venant en regard des pistes du masque.

Dans un mode de réalisation particulier de l'invention, le dispositif ainsi défini est tel que les moyens de positionnement et de guidage du masque permettent d'établir une distance entre le support d'analyse et le point de la piste du masque le plus proche dudit support (correspondant au point le plus bas de la pente), comprise entre 0,1 mm et 0,5 mm, et une distance inférieure à 2 mm, de préférence inférieure ou égale à 1,5 mm au point d'éloignement le plus important entre le masque et le support (correspondant au point le plus haut de la pente).

La distance entre le support d'analyse et le point de la piste du masque le plus proche dudit support est de préférence de 0,5 mm.

Les moyens de positionnement et de guidage du masque selon l'invention peuvent être tous moyens appropriés, le cas échéant présents dans un appareil de réalisation d'électrophorèse. Un limiteur de course peut par exemple être une butée.

Les moyens de guidage comprennent avantageusement un limiteur de course permettant de délimiter la course de déplacement du masque.

Les moyens de positionnement et de guidage peuvent dans un mode de réalisation particulier de l'invention, permettre le déplacement automatisé du masque le long du support d'analyse. Néanmoins, le masque selon l'invention peut sans difficulté être déplacé manuellement, de façon à couvrir par le balayage du support d'analyse, le cas échéant au moyen de plusieurs déplacements allers et retours, la totalité des zones déterminées, avec les réactifs contenus dans le masque.

La demande décrit également un procédé de dépôt et d'étalement d'un ou plusieurs réactifs sur un support d'analyse comprenant des échantillons biologiques, ce procédé comprenant les étapes de :
- positionnement d'un masque ci-dessus défini ou d'un dispositif ci-dessus défini, à proximité du support d'analyse,
- chargement du ou des réactifs sur le masque de façon à permettre le dépôt du ou des réactifs sur le support d'analyse, leur maintien par capillarité entre ledit support et la ou les pistes dudit masque ;
- déplacement du masque par balayage du support d'analyse de façon à permettre l'étalement du ou des réactifs sur le support d'analyse au niveau de zones délimitées dudit support, le ou les réactifs étant étalés en quantité suffisante pour permettre leur interaction avec les constituants des échantillons biologiques présents sur ledit support d'analyse.

Lorsque les pistes du masque comportent une partie en pente, le déplacement du masque est effectué dans un plan horizontal par rapport au plan du support d'analyse au-dessus du support et parallèlement au support.

Lorsque les pistes du masque ne comportent pas de pente et sont donc positionnées de façon inclinées par rapport au support d'analyse pour réaliser une pente, le déplacement du masque est effectué parallèlement au plan du support d'analyse lui même en position horizontale si le masque est incliné.

Lorsque le masque est positionné à proximité du support d'analyse et dès que les réactifs sont venus en contact avec le support d'analyse, on peut procéder immédiatement à son déplacement par balayage au dessus dudit support d'analyse.

Lorsque les réactifs sont étalés sur les zones déterminées du support d'analyse, par exemple sur les zones correspondant aux pistes de migration électrophorétique des échantillons biologiques, ces zones constituent des zones d'incubation desdits réactifs avec les constituants des échantillons.

Un avantage de l'utilisation du masque selon l'invention réside dans le fait que lorsque l'étalement est terminé, le masque peut être immédiatement éloigné des zones délimitées constituant les zones d'incubation du support d'analyse, la quantité de réactif chargée dans le masque étant épuisée.

Un autre avantage lié à l'utilisation du masque selon l'invention est de permettre un étalement uniforme des réactifs sur les zones d'incubation.

Avantageusement, on utilise une quantité de réactif en excès, par rapport à la quantité nécessaire pour recouvrir les zones du support d'analyse au niveau desquelles doit intervenir l'incubation entre les constituants de l'échantillon et les réactifs. Une quantité de réactif en excès est une quantité supérieure à celle répartie par un passage simple (un balayage), à une vitesse de balayage du support d'analyse d'environ 2 cm/s.

La quantité de réactif laissée sur-le support d'analyse par unité de surface balayée, dépend de la surface et notamment de la longueur de balayage. Elle augmente quand la vitesse de balayage diminue.

La vitesse de déplacement du masque par rapport au support d'analyse est normalement comprise entre 0,5 et 2 cm/s.

A titre d'exemple, pour étaler des réactifs sur un support d'électrophorèse ayant une largeur de 8 cm déterminée entre l'anode et la cathode (correspondant à la longueur de balayage), deux déplacements comportant chacun un aller et retour peuvent être effectués, chaque déplacement pouvant être réalisé en 3 secondes environ. Dans ce cas, on peut déposer une quantité de réactif par piste comprise entre 6 µl et 10 µl.

A vitesse lente, c'est à dire à environ à 0,5 cm/s et pour une largeur de piste du masque de 2,5 mm, on épuise par exemple un réactif en quantité de 3-4 µl introduits sous la piste, après 70 mm de course.

Ainsi, pour des pistes de 2,5 mm de large et une course de balayage du masque par rapport au support d'analyse de 70 mm, on utilisera avantageusement un volume de réactif d'environ 8 à 10 µl/piste.

Ainsi, quand le premier balayage aura été réalisé, il restera encore du réactif sous la piste même si le déplacement est lent de 0,5 cm/s et a fortiori si le déplacement est à vitesse moyenne de 2 cm/s.

D'autres balayages seront nécessaires pour épuiser la totalité des réactifs introduits. Le nombre de balayages peut varier en fonction du volume de réactif introduit dans chaque piste.

En pratique, le volume de chaque réactif mis en oeuvre est tel que 4 balayages sont suffisants pour épuiser le réactif.

Avec 10 µl chargés au niveau de chaque ouverture, le masque est par exemple soumis à 2 allers et retours pour une longueur de balayage de 70 mm. Une fois tous les réactifs répartis en surface du gel après ces 4 passages, le masque est retiré sans qu'il y ait de risque d'étalement inopiné et la phase d'incubation proprement dite débute.

Selon un mode de réalisation particulièrement avantageux de l'invention, on procède à deux balayages (un aller et retour) pour étaler les réactifs sur le support d'analyse. Même si après les balayages, une faible quantité de réactif reste sur le support d'analyse, il n'est pas nécessaire de l'enlever avant l'incubation. Les conditions de mise en oeuvre du masque permettent de réaliser un étalement uniforme.

Si on diminue la longueur de balayage, on peut avantageusement diminuer la quantité de réactif étalé par piste.

On a indiqué ci-dessus que le masque utilisé pour la mise en oeuvre du procédé de dépôt et d'étalement d'un ou plusieurs réactifs sur un support d'analyse est avantageusement (cependant pas nécessairement) un masque dans lequel les pistes destinées au fixateur capable de fixer les constituants des échantillons biologiques pour réaliser un profil témoin sont décalées par rapport aux autres pistes selon ce qui a été décrit plus haut.

Ce décalage entre la piste du fixateur et par exemple les pistes destinées aux antisérums spécifiques évite l'interaction entre les réactifs lors de leur dépôt sur le support d'analyse. Ce décalage se justifie en particulier lorsque tous les réactifs sont chargés au niveau du masque et déposés ensemble.

Alternativement, par exemple lorsque les pistes destinées au fixateur ne sont pas décalées, on procédera à un chargement du masque en deux fois, de façon à déposer dans un premier temps, les antisérums et dans un second temps, le fixateur. Ce chargement en deux fois peut être réalisé alternativement en procédant d'abord au chargement et au dépôt du fixateur puis au chargement et au dépôt des réactifs spécifiques.

Alternativement encore, le chargement peut être réalisé en une fois, si les conditions de dépôt des réactifs sur le support d'analyse sont telles qu'elles ne conduisent pas à des interactions entre les réactifs, en particulier entre les réactifs spécifiques et le fixateur s'il est présent.

Lorsque le masque est tel qu'il doit être utilisé en position inclinée pour réaliser une pente au niveau des pistes, par rapport au support d'analyse, les pistes ne sont pas décalées les unes par rapport aux autres mais le chargement des réactifs dont on veut éviter qu'ils interagissent (fixateur et antisérums par exemple) est fait soit en deux temps : le réactif chargé dans un premier temps est étalé par balayage avant le chargement du réactif (par exemple de l'antisérum) chargé dans un deuxième temps, soit dans des conditions qui permettent d'éviter les interactions gênantes lors du dépôt des réactifs sur le support d'analyse.

Le procédé de dépôt et d'étalement de réactif selon l'invention est selon un mode de réalisation particulier, tel que le chargement du masque au moyen du ou des réactifs s'effectue en dehors de la zone de la surface du support d'analyse comprenant les échantillons biologiques.

En effet, pendant ce remplissage qui nécessite un certain temps (30 secondes à 2 minutes), la zone du support d'analyse recouverte par les réactifs à ce niveau est plus large que la piste elle-même par suite d'un phénomène de diffusion. Cette diffusion pourrait entraîner un élargissement anormal du profil, révélé après l'incubation du réactif avec les constituants de l'échantillon biologique, si ce remplissage se faisait à la verticale d'une zone comportant un profil des constituants des échantillons devant être révélés.

Si le chargement du masque est réalisé en dehors des zones du support d'analyse comportant les constituants des échantillons, cet inconvénient résultant de la diffusion des réactifs de la zone de dépôt ne se produit pas.

Ainsi, lorsque la taille de la surface du support d'analyse le permet, ledit chargement peut être effectué dans la partie anodique au delà de la zone dans laquelle se situent les profils de migration électrophorétique desdits échantillons. Le chargement peut alternativement être effectué dans la partie cathodique au delà de la zone des profils de migration électrophorétique des échantillons.

Lorsque la taille de la surface du support d'analyse ne permet pas un tel dépôt en dehors de la zone contenant les profils de migration électrophorétique des échantillons, le chargement du masque peut être réalisé en dehors de la surface du support, par exemple sur une feuille de plastique fin, cette feuille venant en appui sur le support d'analyse et dans le plan de la surface de ce support mais débordant de cette surface.

L'invention a aussi pour objet un procédé de dépôt et d'étalement d'un ou plusieurs réactifs sur un support d'analyse comprenant des échantillons biologiques, ce procédé comprenant les étapes de :
- chargement du ou des réactifs sur le masque de façon à permettre le dépôt du ou des réactifs sur le support d'analyse, leur maintien par capillarité entre ledit support et la pente de la ou des pistes dudit masque ;
- positionnement d'un masque ci-dessus défini ou d'un dispositif ci-dessus défini, à proximité du support d'analyse de façon à permettre le dépôt du ou des réactifs sur le support d'analyse et leur maintien par capillarité entre ledit support et la pente de la ou des pistes dudit masque ;
- déplacement du masque de façon à réaliser une balayage de la surface du support d'analyse de façon à permettre l'étalement du ou des réactifs sur le support d'analyse au niveau de zones d'incubation, correspondant à des zones délimitées dudit support, le ou les réactifs étant étalés en quantité suffisante pour permettre leur interaction avec les constituants des échantillons biologiques présents sur ledit support d'analyse.

Les caractéristiques indiquées précédemment pour la réalisation du procédé de dépôt et d'étalement sont applicables ici.

Selon ce mode d'utilisation du masque mobile chargé préalablement à son positionnement au dessus du support d'analyse on a introduit tous les réactifs dans les orifices supérieurs des ouvertures associées à chaque piste du masque qui jouent alors le rôle de réservoir. Ces réactifs y sont maintenus malgré la présence de l'orifice inférieur de chaque ouverture, du fait des forces de capillarité.

Dans les configurations décrites ci-dessus, quand on charge le masque au-dessus du support d'analyse, le masque étant déjà placé à la distance requise (environ 0,5 mm pour le point le plus bas) dudit support, on introduit directement les réactifs entre les pistes du masque et le support d'analyse.

Pour cela, pendant la phase d'expulsion du réactif qui a été prélevé dans l'embout de la pipette, on assure l'étanchéité entre l'extrémité de cet embout et l'orifice supérieur du masque, par exemple en maintenant la pipette en position verticale avec l'embout légèrement appuyé au fond de l'ouverture du masque au niveau de la partie conique voisine de l'orifice inférieur de l'ouverture. Lorsque cette partie conique inférieure est prolongée par une partie cylindrique, cette dernière a un diamètre (par exemple 0,8 mm) qui ne permet pas le passage de l'embout de la pipette. Cela assure la sortie « forcée » du réactif par l'orifice inférieure de l'ouverture associée à la piste, la goutte qui perle de cet orifice inférieur venant en contact avec le support d'analyse situé à proximité (0,5 mm) et venant s'étaler par capillarité entre la piste et le support. Dans le cas où l'étanchéité embout pipette-orifice supérieur n'est pas obtenue, le réactif reste dans l'orifice supérieur et ne descend pas sur le support d'analyse.

C'est également ce qui se produit dans le cas où l'on assure l'étanchéité mais sans qu'il y ait à proximité de l'orifice inférieur la surface du support d'analyse, c'est à dire quand le chargement est réalisé en dehors du support d'analyse.

Dans ces conditions, la goutte qui a perlé (mais qui du fait de son très faible volume 10 à 15 µl n'est pas tombée) et est restée accrochée par capillarité au voisinage de l'orifice inférieur, remonte dans le puits constitué par l'orifice supérieur quand le « contact embout-orifice supérieur » est supprimé par écartement de la pipette.

Ce mode d'utilisation particulier du masque avec chargement préalable à son positionnement au dessus du support d'analyse présente l'avantage de pouvoir être réalisé de façon automatique par exemple au moyen de l'automate Hydraplus SEBIA supprimant ainsi tout pipetage manuel aboutissant à une simplification accrue du remplissage du masque.

Le masque chargé avec les réactifs répartis dans les orifices supérieurs jouant le rôle de réservoir peut être conservé en chambre humide pendant une durée variant de quelques minutes à plusieurs heures, avant utilisation.

Pour amener les réactifs chargés dans le masque à être déposés sur le support d'analyse, différents moyens sont envisageables.

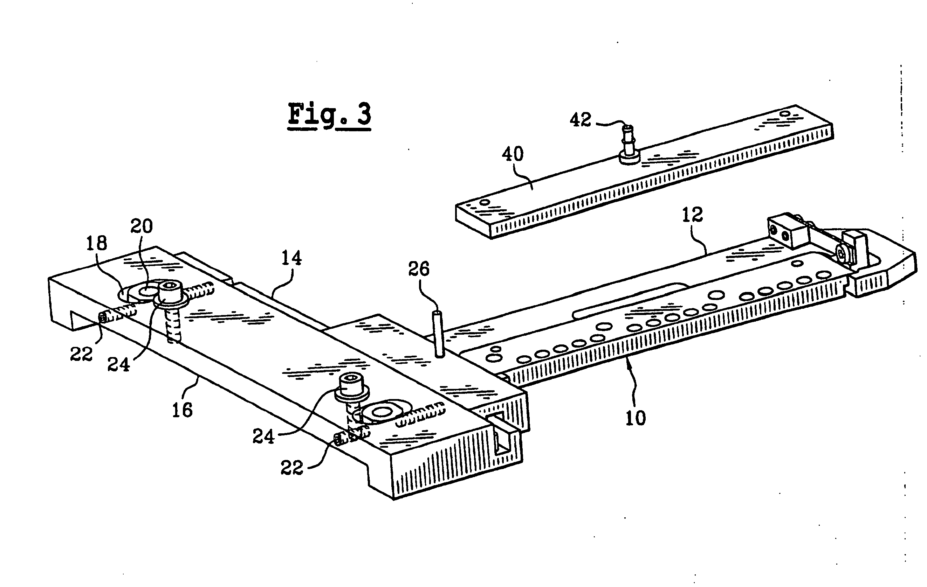

Selon un premier mode de réalisation, l'ensemble masque et porte masque (le porte masque constituant un moyen de positionnement du masque) chargé de différents réactifs est positionné au-dessus du support d'analyse par accroche sur le rail de guidage et amené en butée en position anodique. L'ensemble du masque est recouvert par une petite chambre (figure 3) englobant l'ensemble des orifices supérieurs et prenant appui à la périphérie du masque (appui étanche surface plane contre surface plane). Cette chambre dispose d'un embout par lequel on injecte rapidement (au moyen d'une seringue par exemple) un petit volume d'air compris entre 50 et 200 µl. Cette augmentation de pression dans la chambre étanche fait perler chacun des réactifs en dessous des pistes du masque et ces réactifs viennent toucher le gel. A la suite de ce contact avec le support d'analyse, ils s'étalent simultanément par capillarité entre les pistes du masque et le support d'analyse. On procède alors au balayage de la surface du support d'analyse.

Selon un autre mode de réalisation, l'ensemble masque et porte masque préalablement chargé est positionné au-dessus du support d'analyse par accroche du porte masque sur le rail de guidage et est amené en butée en position anodique.

La descente des réactifs sur le support d'analyse à partir des orifices supérieurs du masque qui jouent le rôle de réservoir peut être obtenue en introduisant verticalement dans chacun des orifices supérieurs une tige cylindrique de diamètre inférieur à celui de l'orifice inférieur du masque (par exemple 0,5 mm) constitué d'un matériau de nature hydrophile (par exemple acier inox) jusqu'à ce que cette tige vienne en contact avec le support d'analyse.

Cette tige permet d'établir une jonction entre le liquide introduit dans l'orifice supérieur et le support d'analyse. La totalité du liquide introduit descend alors par capillarité le long de la tige et se répartit entre la piste et le support d'analyse.

La descente simultanée de tous les réactifs sur le support d'analyse peut être obtenue en introduisant verticalement et simultanément une tige dans chacun des orifices supérieurs du masque, ces tiges de même longueur (5 à 10 mm) étant rendues solidaires par exemple par enfichage dans une plaque de plexiglass rectangulaire de même dimension que le masque et avec un géométrie telle qu'elle reproduise exactement la disposition des orifices du masque. Quand tous les réactifs se sont étalés entre les pistes du masque et le support d'analyse, on retire la plaque de plexiglas munie de ces tiges et on procède au balayage du masque à la surface du gel.

La demande décrit encore un procédé permettant de faire descendre simultanément sur le gel tous les réactifs, gui consiste après avoir positionné le masque en butée anodique au-dessus du support d'analyse, à lui communiquer une impulsion mécanique. Cette impulsion peut par exemple être obtenue par encliquetage dans le support masque, du masque chargé.

Cette impulsion permet de projeter par inertie une goutte de réactif jusqu'ici maintenue par capillarité dans le réservoir du masque, sur le support d'analyse et d'établir ainsi une jonction entre le point le plus bas des pistes du masque au niveau de l'orifice inférieur des pistes et le support d'analyse pour que tous les réactifs situés dans les réservoirs s'étalent par capillarité entre les pistes du masque et le support d'analyse. On peut alors procéder au balayage de la surface du gel.

Encore selon un mode avantageux de réalisation lorsque le masque est chargé en dehors du support d'analyse, après répartition des réactifs dans les orifices supérieurs et installation du masque au dessus du support d'analyse, on amène brièvement le masque en contact avec le support d'analyse, au point le plus bas de la pente des pistes. Ce contact, qui doit être passager a pour objet de permettre alors la descente de tous les réactifs sur le support d'analyse, avant de procéder à l'étalement.

Une fois le masque chargé et en position d'utilisation selon l'un des modes de réalisation ci-dessus, c'est à dire lorsque chaque piste a reçu une quantité de réactif déterminée, le masque est déplacé parallèlement au support d'analyse grâce aux moyens de guidages, dans le sens de la migration électrophorétique. Ce déplacement a pour conséquence d'entraîner le liquide situé entre le masque et le support d'analyse, par balayage de la surface du support d'analyse venant en regard des pistes du masque. Le liquide maintenu par capillarité entre le masque et le support d'analyse est entraîné en particulier grâce à la pente des pistes qui permet de rassembler le réactif vers so point le plus bas pendant le déplacement et une certaine quantité de réactif est alors déposée sur le support d'analyse dans lequel elle pénètre et demeure. Au fur et à mesure du balayage réalisé, le volume de liquide contenu sous la piste est donc consommé par pénétration dans le support d'analyse et diminue.

L'invention a aussi pour objet un procédé de dépôt et d'étalement de réactifs sur un support d'analyse, dans lequel l'étape de chargement du masque avec le ou les réactifs est automatisée.

Dans un autre mode de réalisation préféré de l'invention, l'étape de déplacement du masque pour la réalisation du balayage du support d'analyse est automatisée.

Le procédé selon l'invention est avantageusement mis en oeuvre pour la détection de constituants d'échantillons biologiques, préalablement séparés par migration électrophorétique, cette détection pouvant notamment faire intervenir la technique d'immunofixation, les réactifs étant dans ce cas des antisérums spécifiques et de façon préférée un fixateur pour réaliser un profil électrophorétique témoin.

Un tel procédé selon l'invention peut être mis en oeuvre dans les conditions habituelles de la réalisation des techniques d'électrophorèse et d'immunofixation. Ainsi, les réactifs utilisés sont les réactifs habituellement mis en oeuvre, ces réactifs étant cependant avantageusement utilisés en quantité inférieure dans le cadre de l'invention, par rapport aux quantités habituellement utilisées.

Avantageusement dans le cadre de l'invention, les procédés définis précédemment permettent avec un masque de 18, 12, ou 6 pistes l'analyse simultanée respectivement de 3n, 2n, n échantillons biologiques, n étant un nombre entier représentant le nombre de rangées de dépôts. De préférence, n est égal à 2, 3, ou 4.

Cependant, il n'existe pas de limite de principe au nombre de pistes du masque.

Du fait de la structure du masque et des modalités de son utilisation, la quantité de chaque réactif chargée au niveau de chaque piste du masque, c'est à dire introduite dans chaque ouverture de chaque piste et maintenue par capillarité au-dessous de chaque piste peut avantageusement être réduite et par exemple être inférieure à 15µl/piste. De préférence, cette quantité est inférieure ou égale à 10 µl/piste.

Lorsque les réactifs sont déposés sur le support d'analyse, ils sont sous forme liquide. L'invention a donc pour objet l'utilisation des réactifs liquides pour charger le masque. L'invention a aussi pour objet l'utilisation d'un masque chargé avec des réactifs liquides, le masque étant ensuite lyophilisé de façon à ce que les réactifs contenus dans les ouvertures du masque soient lyophilisés, jusqu'à l'utilisation du masque où, pour l'étape de dépôt, les réactifs sont sous forme de solutions.

L'invention a en outre pour objet un kit comprenant :
- au moins un masque ci-dessus défini,
- au moins un support d'analyse, notamment un support d'électrophorèse.

Un tel kit peut en outre comprendre :
- des réactifs pour l'immunofixation des constituants des échantillons séparés par électrophorèse,
- un fixateur pour la fixation pour chaque échantillon de l'ensemble des constituants séparés par électrophorèse.

Le kit peut aussi comprendre au moins un peigne pour le dépôt des échantillons sur le support d'analyse.

Si le masque doit être chargé en dehors du support d'électrophorèse, le kit peut comprendre des moyens, par exemple décrits précédemment, pour faire descendre les réactifs des ouvertures comprises dans le masque sur le support d'analyse.

Un tel kit est de façon préférée, adapté à la séparation simultanée de 9 voire 12 échantillons sur chaque support d'électrophorèse, au moyen d'un masque à 18 pistes.

De préférence un tel kit permet la séparation simultanée de 18 échantillons sur chaque support d'électrophorèse.

Un kit selon l'invention peut également contenir des indications relatives à l'utilisation du masque de l'invention, par exemple sous la forme d'une notice d'utilisation comprenant des informations sur les quantités de réactifs à charger sur le masque et/ou sur les conditions de déplacement du masque telles que la vitesse de balayage ou le nombre de balayages conseillés.

Le cas échéant, dans un mode de réalisation particulier du kit de l'invention, le masque n'est pas accompagné du support d'analyse, qui peut être obtenu indépendamment.

Le masque selon l'invention est avantageusement chargé en réactifs.

Par ailleurs, l'invention a pour objet un support d'électrophorèse pour la séparation d'au moins 9 échantillons biologiques disposés selon 3 rangées de 3 échantillons, en vue de leur immunofixation, ledit support comprenant au moins 18 pistes de migration, lesdites pistes étant espacées les unes des autres d'une distance de 2 mm et ayant une largeur de 3 mm, et la longueur totale des pistes de migration étant d'au plus 63 mm.

D'autres caractéristiques, détails et avantages de l'invention apparaîtront à la lecture de la description qui suit, faite à titre d'exemple en référence aux dessins annexés, dans lesquels :
- la figure 1 est une vue schématique en perspective, de dessous, d'un dispositif porte-masque selon l'invention ;
- la figure 2 est une vue schématique en perspective, de dessus, de ce dispositif ;
- La figure 3 est une vue schématique de dessus en perspective, de ce dispositif et d'un couvercle associé ;
- Les figures 4 et 5 sont des vues de dessous et de côté respectivement du masque selon l'invention ;
- La figure 6 est une vue en coupe selon la ligne VI-VI de la figure 4 ;
- Les figures 7 et 8 sont des vues schématiques en perspective de deux variantes de réalisation ;
- Les figures .9 à 17 illustrent un autre mode de réalisation du masque dans lequel les pistes sont alignées selon une seule rangée.

### Figure 9

Vue du dessus du guide permettant de positionner le masque au dessus du gel d'électrophorèse et de permettre le balayage du masque au dessus du gel selon une direction et une course déterminée.

### Figure 10

Vue de dessus et de dessous du chariot. Chariot s'accrochant sur le rail (14) au moyen de la glissière (49). Le chariot possède quatre pieds (30) qui prennent appui sur le plan où est positionné le gel d'électrophorèse de part et d'autre de ce gel. Ces pieds (30) peuvent glisser sur ce plan.

### Figure 11

Vue de dessus et de dessous du porte masque qui reçoit et maintient le masque au moyen des encoches (43) et des ressorts (28).

Le porte masque présente une zone d'appui (46) où l'on exerce une pression pour amener le masque en contact avec le gel.

### Figure 12

Ensemble chariot et porte masque rendus solidaires par l'intermédiaire des deux lames en acier ressort (47) maintenues au moyen de rivets (48). Cet ensemble, une fois accroché sur le rail guide (14) par la glissière (49) permet de maintenir le masque parallèlement au gel et à proximité de celui-ci sans qu'il y ait contact.

Pour faire descendre les réactifs qui ont été chargés dans les puits du masque, on exerce en (46) une pression sur le porte masque. Cela entraîne une flexion des lames ressort (47) et la partie la plus basse des pistes où est situé l'orifice inférieur des puits de remplissage (34) vient en contact avec le gel (un système de butée permet de limiter la course de cette flexion afin de ne pas endommager le gel).
En relâchant la pression en (46), le masque remonte et reprend la position d'origine et l'ensemble des réactifs contenus dans les puits (36) se répartissent par capillarité entre la partie inférieure des pistes (32) et le gel. On procède alors au balayage au moyen de la poignée (26) en réalisant un aller-retour.

### Figure 13

Montre une vue de dessus de l'ensemble chariot + porte masque + masque.

### Figures 14 à 17

Montrent les vues de dessus et de dessous de divers types de masques.

### Figure 18

Montre l'ensemble guide + masque fixé sur la barre de positionnement (50) sur le plateau d'électrophorèse en positions extrêmes de balayage.

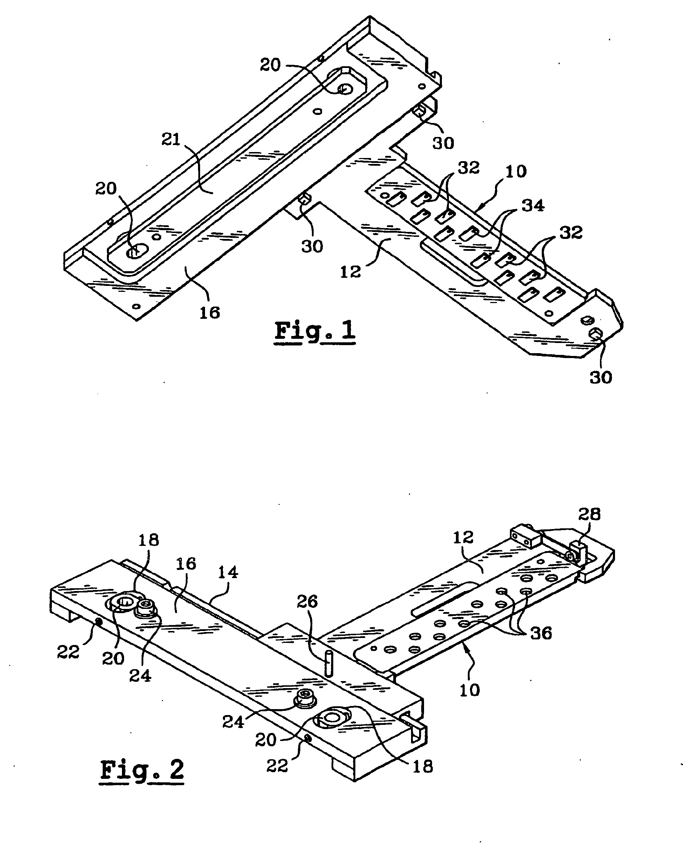

Le dispositif représenté aux figures 1 à 3 comprend un masque 10 selon l'invention, monté de façon amovible sur un bras support 12, sensiblement en forme de C, qui est accroché et guidé en translation sur un rail 14 d'une barrette 16 de positionnement et de fixation sur un plateau (non représenté) qui porte le support d'analyse (tel par exemple qu'un gel d'agarose).

La barrette 16 s'étend sur un bord de ce plateau parallèlement à la direction de migration électrophorétique et comporte deux lumières transversales 18 dans lesquelles s'engagent des plots évidés 20 portés par une autre barrette 21 solidaire du bord du plateau, les évidements des plots 20 s'emboîtant sur des tétons émergeant du plateau de migration (non représentés) et associés à des vis 22 de réglage de position transversale de la barrette 16 et donc du masque 10 par rapport au plateau et au support d'analyse. La barrette comprend de plus des vis 24 permettant de bloquer la barrette après son réglage.

Le rail 14 s'étend parallèlement à la direction de migration électrophorétique et est engagé dans une rainure correspondante d'une extrémité du bras 16. Cette extrémité porte une poignée ou une tige 26 de déplacement en translation sur le rail 14 dans un sens et dans l'autre.

En variante, des moyens motorisés peuvent être prévus sur la barrette 16 pour le déplacement automatisé du bras 12, par exemple un micromoteur électrique dont l'arbre de sortie porte une roue dentée, ou une poulie, coopérant avec une chaîne, ou une courroie respectivement, reliée au bras 12.

Le masque 10 est fixé sur le bras 12 par tout moyen approprié, par exemple par encliquetage élastique comme représenté en 28 et est formé d'une plaque plane allongée sensiblement rectangulaire, qui s'étend transversalement, c'est à dire perpendiculairement, à la direction de migration électrophorétique.

Cette plaque est maintenue au-dessus du support d'analyse, à distance prédéterminée de celui-ci, par des plots ou des cales 30 formés ou fixés en saillie sur la face inférieure du bras 12 et qui sont destinés à reposer sur les bords du plateau précité.

La face inférieure du masque 10 comporte une série de pistes (ou rampes) obliques 32, parallèles entre elles et agencées en deux rangées transversales dans lesquelles elles sont décalées en quinconce, dans l'exemple représenté. Ces pistes (ou rampes) 32 s'étendent parallèlement à la direction de migration électrophorétique et sont toutes inclinées dans le même sens. Leur extrémité la plus basse comporte un orifice 34 de dépôt de réactif sur le support d'analyse. Cet orifice 34 est l'orifice inférieur d'un conduit ou passage traversant le masque 10 sur toute son épaisseur et débouchant sur la face supérieure du masque par un orifice 36 ayant un diamètre nettement plus grand que celui de l'orifice inférieur 34.

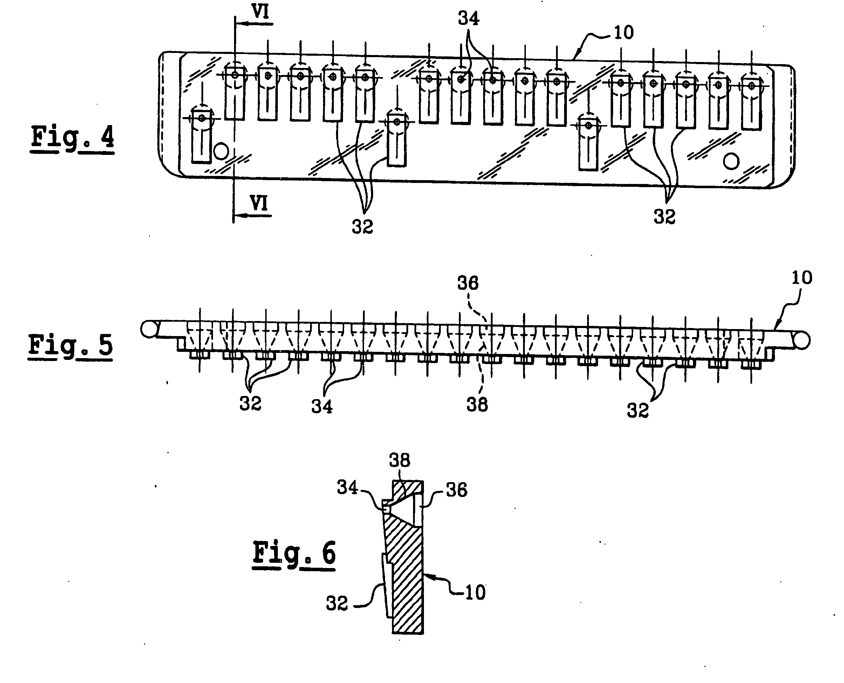

Dans un mode de réalisation de l'invention représenté aux figures 4 à 6, ces orifices 34 et 36 sont les extrémités de petits conduits cylindriques à section circulaire reliés l'un à l'autre par un conduit tronconique 38.

Le porte masque 12 avec le masque 10 encliqueté étant accroché sur le rail de guidage 14, un réglage transversal au moyen des vis 22 permet d'amener les rampes du masque à la verticale des pistes de migration des échantillons qui peuvent être visualisées par addition aux échantillons déposés d'un colorant approprié tel le bleu de bromophenol.

Le balayage du masque au dessus du support d'analyse se fait manuellement au moyen de la poignée 26 ou des moyens motorisés précités.

Comme représenté schématiquement en figure 3, un couvercle 40 peut être posé sur le masque 10 et fixé à celui-ci pour fermer de façon sensiblement étanche les conduits 34, 36, 38 formés dans le masque 10. Une tubulure 42 du couvercle 40 débouche au-dessus des orifices supérieurs 36 de ces conduits et permet l'injection d'une faible quantité d'air entre le masque et le couvercle, pour exercer une pression sur les réactifs contenus dans les conduits du masque et les faire descendre dans les conduits pour les amener en contact du support d'analyse.

On peut ainsi déposer les réactifs dans les conduits du masque 10, fermer ces conduits de façon sensiblement étanche par le couvercle 40, transporter l'ensemble masque 10 - couvercle 40 et le poser au-dessus du support d'analyse, avant d'utiliser les réactifs.

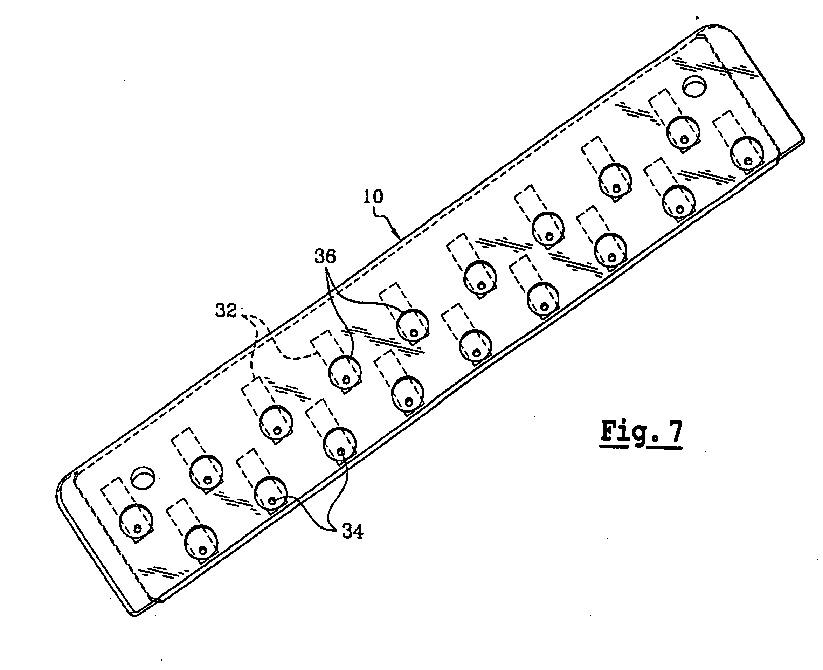

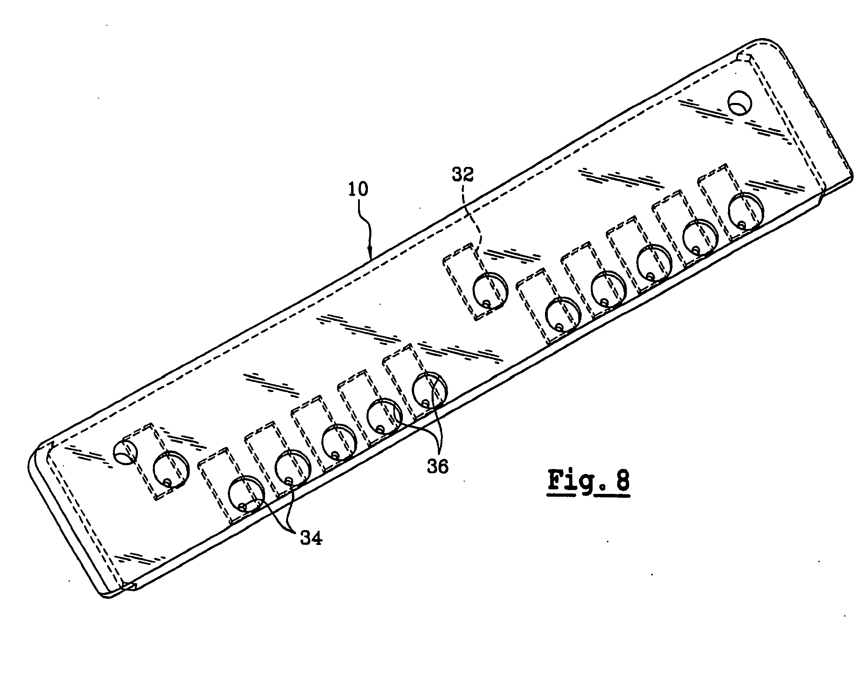

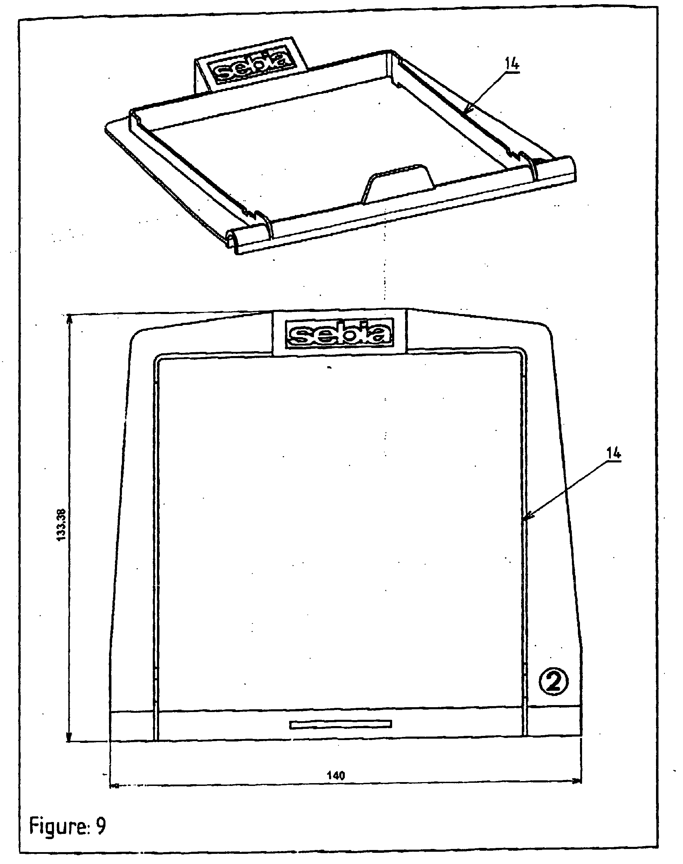

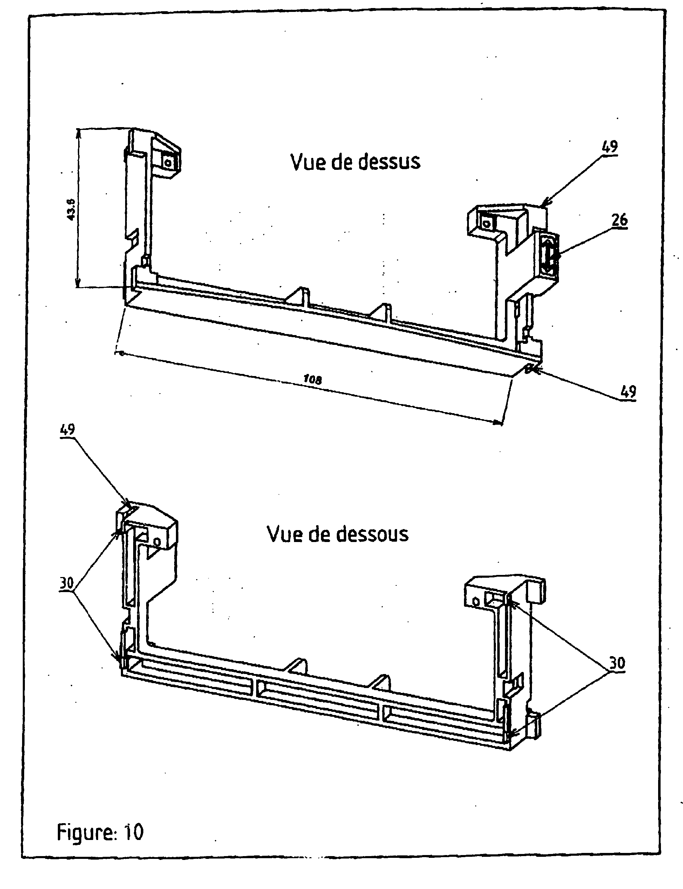

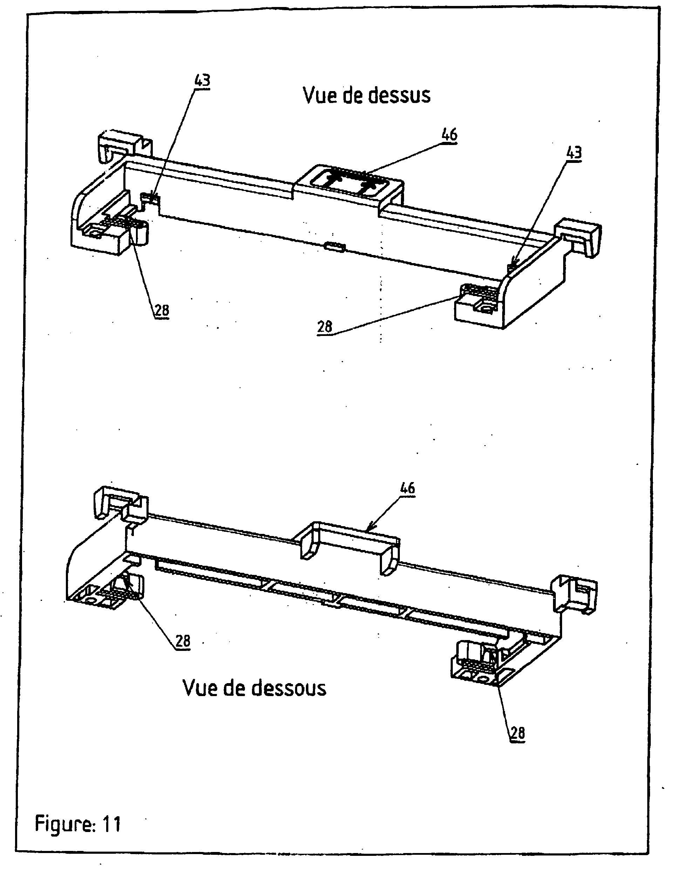

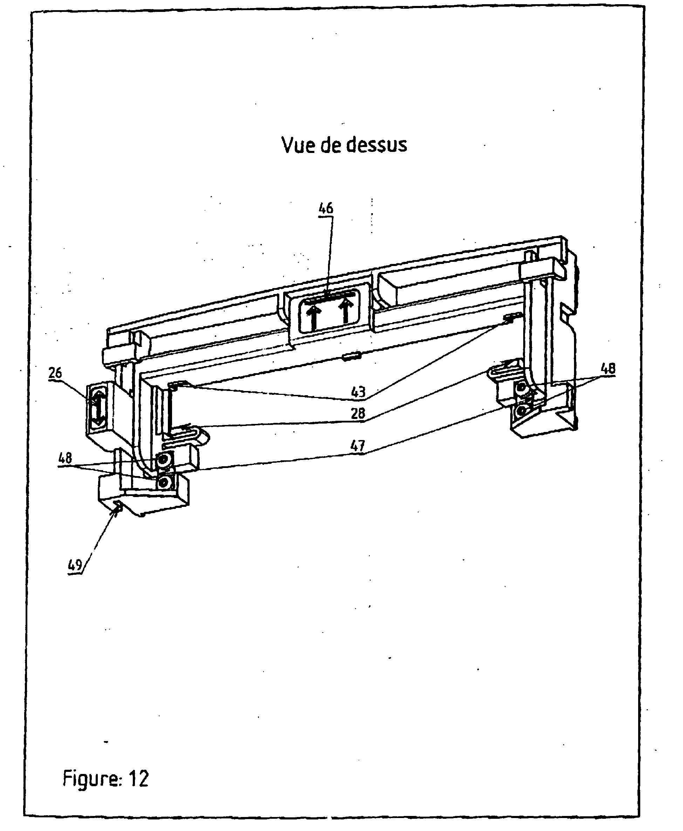

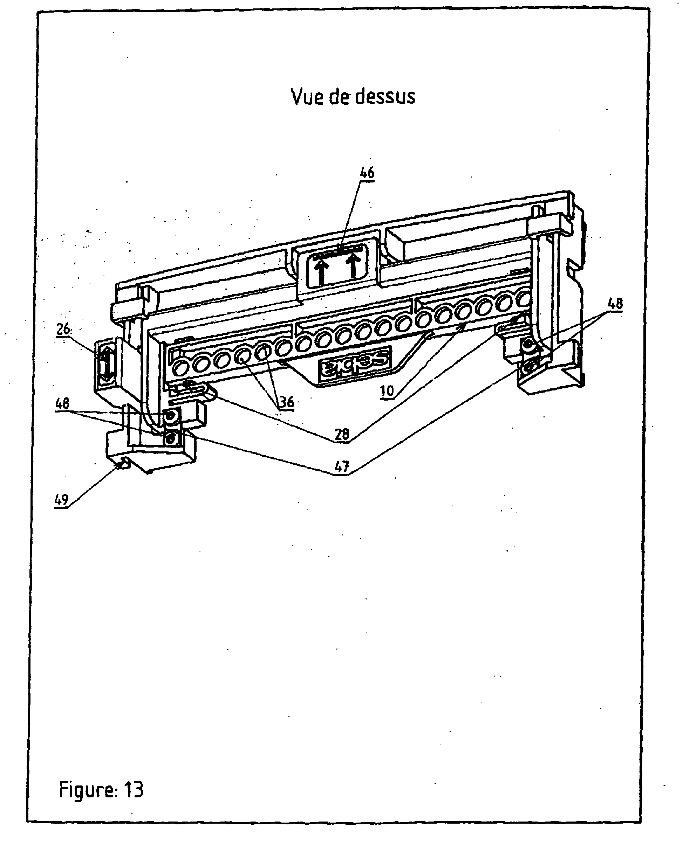

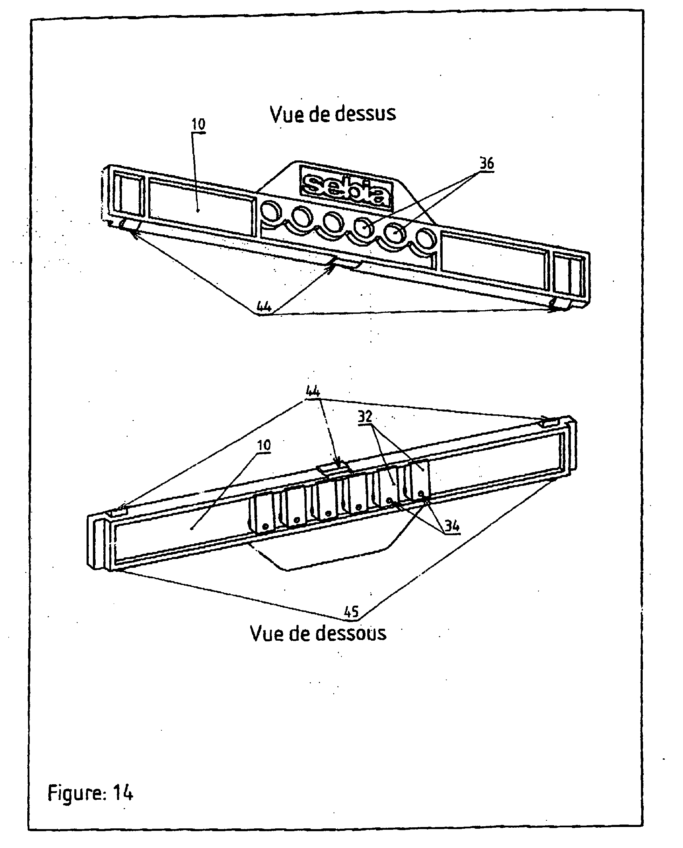

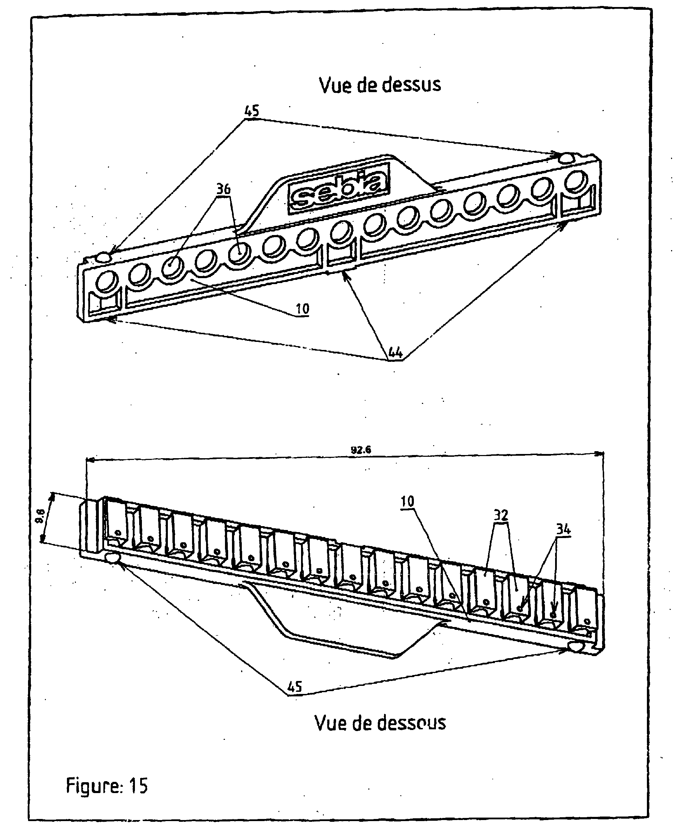

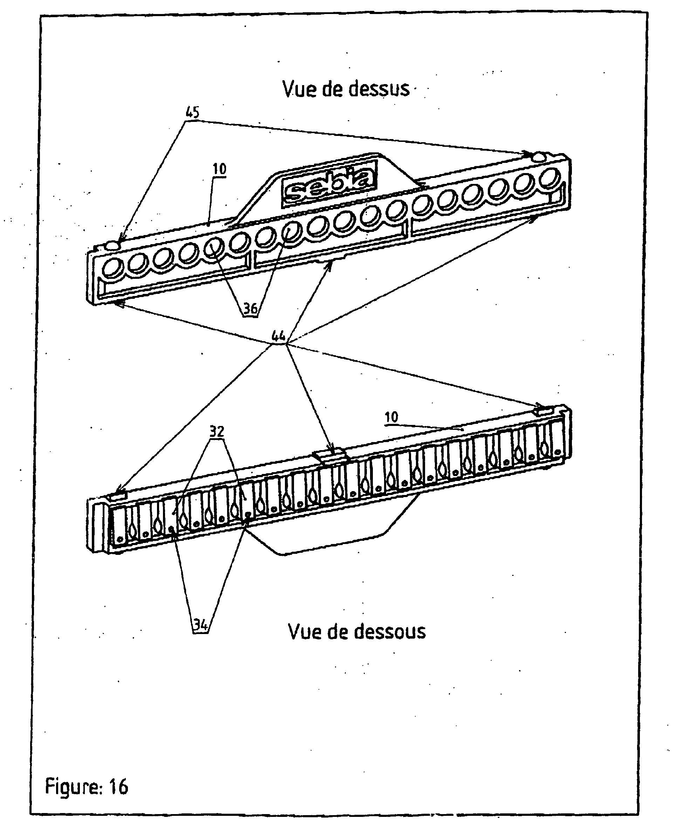

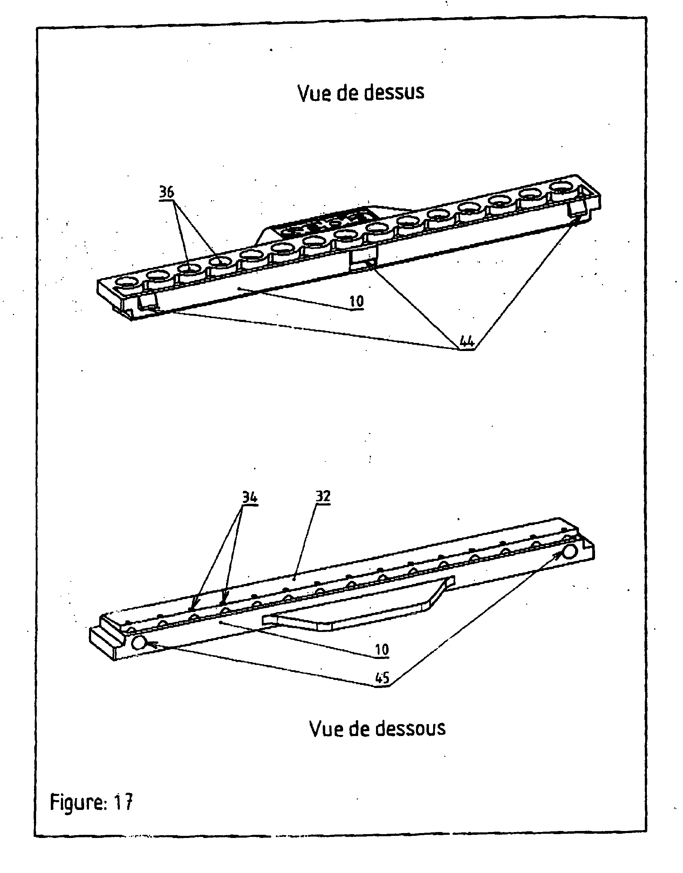

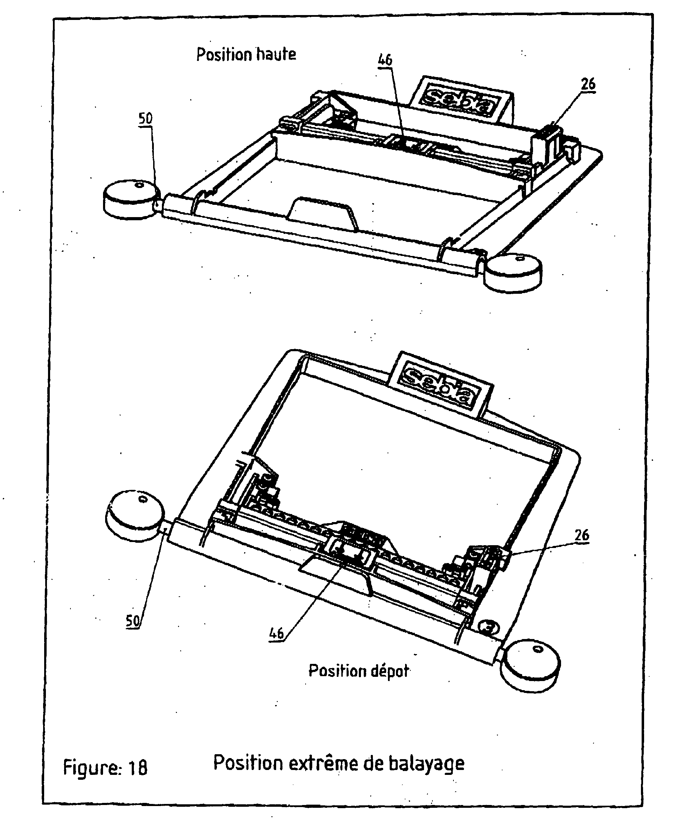

On a représenté schématiquement aux figures 7 et 8 deux variantes de réalisation du masque 10 :
- celui de la figure 7 comprend 18 conduits traversants agencés en deux rangées parallèles et décalés en quinconce d'une rangée à l'autre,
celui de la figure 8 comprend deux groupes de six conduits traversants, les deux groupes étant alignés sur la longueur du masque et comprenant chacun cinq conduits alignés et un conduit décalé.

### - Exemples d'utilisation du masque selon l'invention

### Exemple 1

### Immunofixation simultanée de 9 échantillons au moyen d'un masque mobile à 18 pistes. (Figure 4).

La manipulation a été réalisée au moyen de l'automate d'électrophorèse Hydrasys® de la société SEBIA, sur un gel destiné à l'immunofixation, de dimensions 0,7 x 83 x 101 mm.

Pour le dépôt des échantillons sur le support d'électrophorèse, on a utilisé les applicateurs (brevets France n° 2 671 290 et Européen 0 493 996) comportant 18 dents de 3 mm espacées de 2 mm. Chaque échantillon a été déposé sur 6 dents consécutives, chaque applicateur permettant donc le dépôt de 3 échantillons différents sur le gel d'électrophorèse et on a utilisé 3 applicateurs pour les 9 échantillons à analyser. Les dépôts ont été réalisés sur le gel au moyen de l'automate Hydrasys®, en 3 rangées parallèles situées respectivement à 18, 38 et 58 mm du bord cathodique du gel. La migration électrophorétique a été réalisée à température contrôlée de 20°C, à puissance constante de 20 W et pendant une durée telle que 31 volt heure soient accumulés.

Une fois la migration terminée, on a installé le masque mobile de l'invention à 18 pistes dont les caractéristiques géométriques sont les suivantes : largeur de piste 2,5 mm, longueur de piste 7 mm, écartement interpistes 2,5 mm, pente des pistes 5°. Une rangée de 3 pistes (destinées au chargement du fixateur) est décalée de 5,5 mm par rapport à la rangée de 15 pistes (destinées au chargement des antisérums).

Les évidements des plots 20 du rail de guidage ont été positionnés, sur les deux tétons portés par le plateau de migration de l'automate l'Hydrasys®.

Le masque à 18 pistes a été encliqueté dans le porte-masque 12 qui était lui-même accroché au rail de guidage. Le masque et son porte-masque ont été amenés en butée en position haute c'est à dire du côté anodique du gel. Un colorant, le bleu de bromophénol, incorporé aux échantillons déposés sur le gel, permettait de visualiser la position des pistes électrophorétiques sur le support d'analyse. Au moyen du réglage transversal 20, 22, les pistes ou rampes du masque ont été amenées à l'aplomb de l'alignement des pistes de migration des échantillons.

On a procédé alors au chargement du masque en introduisant par les ouvertures supérieures des pistes (36) les différents réactifs nécessaires à l'immunofixation, à raison de 10 µl de réactif par piste et selon l'ordre habituel : fixateur, anti IgG, anti IgA, anti IgM, anti kappa et anti lambda. Le fixateur a été introduit sous les 3 pistes décalées côté anodique.

Une fois introduite entre les pistes et le gel, ces 10µl de réactif se sont étalés sous chaque piste sur environ 5-6 mm au-delà des zones, côté anodique, devant être révélées.

Dès que le chargement du masque a été terminé, celui-ci a été déplacé au moyen de la poignée (26) par glissement le long du rail de guidage. Ce balayage a été effectué sans à coup, à une vitesse à peu près constante à partir de la position haute (côté anodique) jusqu'à la position basse (côté cathodique) du gel, réalisant une course de 63 mm. Ce balayage a été réalisé en environ 3 secondes.

Le masque étant arrivé en butée en position cathodique, on a alors réalisé un balayage en sens inverse, dans les mêmes conditions. Ces deux balayages ont été répétés une fois. La totalité des réactifs introduits initialement sous les pistes a alors été déposée sur le gel au-dessus des zones de migration électrophorétique. Le masque a pu être retiré avant la phase d'incubation des réactifs sur le gel réalisée pendant 5 minutes à 20°C.

On a procédé ensuite aux étapes de pompage, séchage, lavage, coloration, décoloration, séchage selon les protocoles habituels utilisés en immunofixation.

### Exemple 2

### Immunofixation simultanée de 12 échantillons au moyen d'un masque mobile à 18 pistes (figure 4).

On a procédé comme dans l'exemple précédent, mais on a chargé 4 applicateurs de 18 dents à raison de 3 échantillons par applicateurs. Au moyen de l'automate Hydrasys®, les dépôts ont été réalisés sur le gel en 4 rangées parallèles situées respectivement à 18, 33, 48, et 63 mm du bord cathodique du gel. La migration a été réalisée pendant 28 Volt heure à puissance constante de 20 W, à 20°C. On a procédé ensuite comme pour l'exemple précédent.

### Exemple 3

### Immunofixation simultanée de 4 échantillons au moyen d'un masque mobile à 12 pistes (figure 8)

On a utilisé pour réaliser les dépôts d'échantillons sur le gel, des applicateurs à 15 dents de 4 mm de large, espacées de 2 mm, les applicateurs étant chargés à raison de 2 échantillons par applicateur (échantillon n° 1 dents 2 à 7 ; échantillon n° 2, dents 9 à 14).

Au moyen de l'automate Hydrasys® les dépôts ont été réalisés sur le gel en 2 rangées parallèles situées respectivement à 23 et 53 mm du bord cathodique du gel.

La migration à température contrôlée de 20°C à puissance constante de 20 W a été poursuivie jusqu'à ce que 42 Volt heure aient été accumulés.

Une fois la migration terminée, on a installé le masque mobile 12 pistes dont les caractéristiques géométriques sont les suivantes : largeur de piste 3,5 mm, longueur de piste 7 mm, espace interpistes 2,5 mm, pente des pistes 5°. Une rangée de 2 pistes (destinées au chargement du fixateur) est décalée de 5,5 mm par rapport à la rangée de 10 pistes (destinées au chargement des antisérums).

Les évidements des plots 20 du rail de guidage ont été positionnés sur les deux tétons situés dans le plan du plateau de migration de l'Hydrasys®. Le masque 12 pistes a été encliqueté dans le porte masque qui a été lui-même accroché à la rainure du rail de guidage, l'ensemble étant amené en butée en position haute.

Au moyen du réglage transversal, les pistes du masque ont été amenées comme décrit dans l'exemple 1) à l'aplomb de l'alignement des pistes de migration électrophorétique des échantillons.

On a procédé alors au chargement du masque en introduisant 14 µl de réactif par piste.

L'étalement des réactifs a été réalisé comme dans les exemples précédents en faisant effectuer 4 balayages au masque.

La totalité des réactifs était alors déposée à la surface du gel et le masque a pu être retiré.

On a poursuivi alors l'incubation et les étapes de pompage, séchage, lavage, coloration, décoloration, séchage ont été effectuées comme habituellement.

### Exemple 4

### Réalisation dune technique 36 IF penta au moyen d'un masque mobile 18 pistes (figure 4).

La technique IF penta est utilisée en routine pour détecter dans les échantillons analysés la présence de paraprotéines sous forme de bandes monoclonales ou oligoclonales de nature immunoglobuline.

Cette technique est réalisée en révélant côte à côte pour chaque échantillon analysé, le profil protéique total et le profil de l'ensemble des immunoglobulines en réalisant une immunofixation par un antisérum pentavalent c'est à dire ayant à la fois les spécificités anti IgG anti IgA anti IgM anti kapa et anti lambda.

La manipulation a été réalisée dans le cadre de cet exemple sur un gel d'agarose destiné à l'immunofixation de dimensions 0,7 x 83 x 101 mm au moyen de l'automate d'électrophorèse Hydrasys® SEBIA. On a utilisé des peignes à 18 dents de 3 mm, espacées de 2 mm. Chaque échantillon à analyser a été déposé en double côte à côte soit 9 échantillons par applicateur.

Pour les 36 échantillons, on a utilisé 4 applicateurs.

Les dépôts sur le gel ont été réalisés au moyen de l'automate Hydrasys® en 4 rangées parallèles situées respectivement à 18, 33, 48 et 63 mm du bord cathodique du gel. La migration a alors été réalisée à température contrôlée de 20 °C sous puissance constante de 20 W jusqu'à accumulation de 28 Volts heure. On a alors installé le masque mobile de l'invention à 18 pistes correspondant à la figure 4.

Ce masque est constitué de 2 rangées de 9 pistes chacune, décalées l'une par rapport à l'autre de 5,5 mm. Les 9 pistes les plus anodiques sont destinées à recevoir le fixateur les 9 autres pistes, l'antisérum pentavalent. Chaque piste a une largeur de 2,5 mm, une longueur de 7 mm, un espace interpiste de 2,5 mm et une pente de 5°.

Le masque a été encliqueté dans le porte masque et amené en butée en position haute côté anodique.

Les réactifs ont été introduits à raison de 10 µl / piste.

On a procédé ensuite selon les étapes décrites aux exemples 1 à 3.

## Revendications

1. Masque, convenant pour le dépôt et l'étalement de réactifs sur un support d'analyse d'échantillons biologiques, comprenant :
- une surface inférieure et une surface supérieure au moins en partie parallèles entre elles, séparées par une distance constituant l'épaisseur du masque,
- une ou plusieurs piste(s), correspondant à des zone(s) délimitée(s), localisées au niveau de la surface inférieure du masque et comprenant un élément en saillie (32) faisant saillie à la surface inférieure du masque, chaque élément en saillie comprenant une partie constituant une pente par rapport à un plan horizontal,
- **caractérisé en ce que** le masque comprend, associée à chaque piste, une ouverture pour le chargement et le dépôt de réactifs, ladite ouverture traversant le masque sur la totalité de son épaisseur depuis un orifice supérieur (36) de chargement à la surface supérieure du masque jusqu'à un orifice inférieur (34) de dépôt, ledit orifice inférieur de dépôt étant situé dans la pente, à proximité du point le plus bas de la pente de la piste,
le masque étant tel que la ou les pistes qu'il comporte permettent le maintien par capillarité entre la piste et la surface du support d'analyse en regard de laquelle serait placé le masque, de réactifs chargés au niveau de chaque ouverture et déposés sur le support d'analyse.

2. Masque selon la revendication 1, convenant pour l'étalement de réactifs sur un support d'analyse d'échantillons biologiques, comprenant :
- une surface inférieure et une surface supérieure au moins en partie parallèles entre elles, séparées par une distance constituant l'épaisseur du masque,
- une ou plusieurs piste(s) comprenant chacune un élément en saillie (32) de forme allongée, émergeant sous la surface inférieure du masque, ledit élément en saillie comprenant une partie constituant une pente par rapport à un plan horizontal,
- associée à chaque piste, une ouverture pour le chargement et le dépôt de réactifs, ladite ouverture traversant le masque sur la totalité de son épaisseur depuis un orifice supérieur (36) de chargement présent à la surface supérieure du masque jusqu'à un orifice inférieur (34) de dépôt, ledit orifice inférieur de dépôt étant situé dans la pente, à proximité du point le plus bas de la pente de la piste,
le masque étant tel que la ou les piste(s) qu'il comporte permettent le maintien par capillarité entre la piste et la surface du support d'analyse en regard de laquelle serait placé le masque, de réactifs chargés au niveau de chaque ouverture et déposés sur le support d'analyse.

3. Masque selon la revendication 1 ou 2, convenant pour l'étalement de réactifs sur un support d'analyse d'échantillons biologiques, comprenant :
- une surface inférieure et une surface supérieure au moins en partie parallèles entre elles, séparées par une distance constituant l'épaisseur du masque,
- une ou plusieurs piste(s) comprenant chacune un élément en saillie (32) émergeant sous la surface inférieure du masque, constitué par une protubérance de forme parallélépipédique tronquée, ledit élément en saillie comprenant une partie constituant une pente par rapport à un plan horizontal,
- associée à chaque piste, une ouverture pour le chargement et le dépôt de réactifs, ladite ouverture traversant le masque sur la totalité de son épaisseur depuis un orifice supérieur (36) de chargement présent à la surface supérieure du masque jusqu'à un orifice inférieur (34) de dépôt, ledit orifice inférieur de dépôt étant situé dans la pente, à proximité du point le plus bas de la pente de la piste,
le masque étant tel que la ou les piste(s) qu'il comporte permettent le maintien par capillarité entre la piste et la surface du support d'analyse en regard de laquelle serait placé le masque, de réactifs chargés au niveau de chaque ouverture et déposés sur le support d'analyse.

4. Masque selon la revendication 2 ou 3, convenant pour l'étalement de réactifs sur un support d'analyse d'échantillons biologiques, **caractérisé en ce que** l'élément en saillie (32) a une surface supérieure coïncidant avec la partie de la surface inférieure du masque dont il émerge, et une surface inférieure séparée de la surface supérieure selon au moins une pente par rapport à un plan horizontal.

5. Masque selon la revendication 1, **caractérisé en ce que** chaque élément en saillie (32) comprend une surface inférieure et une surface supérieure parallèles entre elles et parallèles aux surfaces inférieure et supérieure du masque, la pente de la piste étant réalisée par l'inclinaison du masque par rapport au support d'analyse, en position d'utilisation.

6. Masque selon la revendication 5, **caractérisé en ce que** l'élément en saillie (32) est de forme allongée, notamment de forme parallélépipédique.

7. Masque selon la revendication 4 ou 6, **caractérisé en ce que** le point de plus bas de la pente, situé à proximité de l'orifice inférieur (34) de l'ouverture de chaque piste, est le point le plus proche du support d'analyse en regard et à proximité duquel il est amené en position d'utilisation

8. Masque selon l'une quelconque des revendications 1 à 7, **caractérisé en ce qu'**il est rigide ou rigidifié.

9. Masque selon l'une quelconque des revendications 1 à 8, dans lequel l'ouverture a un volume tel qu'elle est susceptible de constituer un réservoir pour les réactifs chargés.

10. Masque selon l'une quelconque des revendications 1 à 9, dans lequel l'ouverture de chaque piste traverse perpendiculairement l'épaisseur du masque y compris l'épaisseur de l'élément en saillie (32), l'ouverture comportant une partie de forme tronconique (38) terminée par un orifice inférieur (34) de forme cylindrique.

11. Masque selon l'une quelconque des revendications 1 à 10, comprenant plusieurs pistes parallèles entre elles, réparties sur la longueur du masque.

12. Masque selon la revendication 11, comprenant :
- une première série de pistes parallèles entre elles, disposées suivant un premier malignement,
- une deuxième série de pistes parallèles entre elles et parallèles aux pistes de la première série, et formant un deuxième alignement, décalé par rapport au premier alignement.

13. Masque selon la revendication 11, dans lequel les pistes sont organisées en plusieurs groupes se succédant sur la longueur du masque, chaque groupe étant constitué par une piste positionnée de façon décalée par rapport aux autres pistes alignées les unes par rapport aux autres, les pistes étant parallèles entre elles.

14. Masque selon l'une quelconque des revendications 1 à 4 et 8 à 13, dans lequel la longueur de la pente de chaque piste coïncide avec la longueur de cette piste.

15. Masque selon l'une quelconque des revendications 1 à 4 et 8 à 13, dans lequel la piste se prolonge au-delà de la pente.

16. Masque selon l'une quelconque des revendications 1 à 15, dans lequel les pistes permettent le dépôt de chaque réactif en quantité égale à ou comprise entre 4 et 15µl dans l'ouverture du masque et le maintien par capillarité desdits réactifs entre la piste et le support d'analyse, lorsque le masque est amené à proximité du support d'analyse.

17. Masque selon la revendication 16, permettant le dépôt de chaque réactif en quantité égale à ou comprise entre 4 et 15µl dans chaque ouverture du masque et le maintien par capillarité desdits réactifs entre les pistes du masque et le support d'analyse, lorsque le masque est amené à une distance du support d'analyse inférieure ou égale à 2 mm au point du masque le plus éloigné du support d'analyse.

18. Masque selon l'une quelconque des revendications 1 à 17, permettant le dépôt de chaque réactif en quantité égale à ou comprise entre 4 et 15µl dans chaque ouverture du masque et le maintien par capillarité desdits réactifs entre les pistes du masque et le support d'analyse, lorsque le masque est amené à une distance du support d'analyse égale à ou comprise entre 0,1 et 0,5 mm au point du masque le plus proche du support d'analyse.

19. Masque selon l'une quelconque des revendications 1 à 18, dans lequel les pistes sont séparées les unes des autres par un écartement supérieur ou égal à 1,5 mm.

20. Masque selon la revendication 19, dans lequel l'écartement entre les pistes est de 2,5 mm.

21. Masque selon l'une quelconque des revendications 1 à 20, dans lequel les dimensions des pistes et leur écartement sont tels que les réactifs qu'elles maintiennent par capillarité entre le masque et le support d'analyse n'interagissent pas lors du dépôt ou de l'étalement sur ledit support d'analyse.

22. Masque selon l'une quelconque des revendications 1 à 21, dans lequel la largeur des pistes est de 2,5 mm.

23. Masque selon l'une quelconque des revendications 1 à 22, dans lequel chaque piste a une longueur comprise entre 6 et 7 mm.

24. Masque selon l'une quelconque des revendications 1 à 23, dans lequel la piste destinée au fixateur est décalée par rapport à la première piste voisine d'une distance de 5 à 7 mm.

25. Masque selon l'une quelconque des revendications 1 à 24, dans lequel la pente de chaque piste forme un angle compris entre 1° et 10° avec un plan horizontal.

26. Masque selon l'une quelconque des revendications 1 à 25, dans lequel chaque piste a les dimensions suivantes :
- longueur: 3 à 15 mm
- largeur: 1 à 10 mm
- Inclinaison de la pente par rapport à un plan horizontal : 1 à 10°.

27. Masque selon l'une quelconque des revendications 1 à 26 dans lequel chaque piste a les dimensions suivantes :
- longueur : 7 mm
- largeur : 2,5 mm
- angle de la pente par rapport à un plan horizontal : 5°.

28. Masque selon l'une quelconque des revendications 1 à 27 dans lequel l'orifice supérieur de l'ouverture forme un angle de 50°.

29. Masque selon l'une quelconque des revendications 1 à 28, dans lequel l'épaisseur du masque est comprise entre 1 et 10 mm.

30. Masque selon l'une quelconque des revendications 1 à 29, comprenant 6, 12 ou 18 pistes.

31. Masque selon l'une quelconque des revendications 1 à 30 réalisé en un matériau propre à être moulé, et permettant de réaliser une surface lisse.

32. Masque selon l'une quelconque des revendications 1 à 31, associé à des moyens de positionnement destinés à maintenir le masque à proximité de la surface du support d'analyse au voisinage duquel le masque serait amené pour le dépôt et l'étalement de réactifs sur le support d'analyse.

33. Dispositif pour le dépôt et l'étalement d'un ou plusieurs réactifs sur un support d'analyse d'échantillons biologiques comprenant :
a) un masque (10) selon l'une quelconque des revendications 1 à 32,
b) des moyens (12, 14, 16) de positionnement et de guidage du masque permettant le positionnement du masque de façon telle que le masque est maintenu à proximité de la surface du support d'analyse et permettant le guidage du masque par balayage de la surface du support d'analyse, dans un plan horizontal parallèle à la surface dudit support ou incliné par rapport à la surface dudit support, pour permettre le dépôt et l'étalement des réactifs sur chacune des zones déterminées du support d'analyse venant en regard des pistes du masque.

34. Dispositif selon la revendication 33, dans lequel les moyens de positionnement et de guidage du masque (10) permettent d'établir une distance entre le support d'analyse et le point de la piste du masque le plus proche dudit support comprise entre 0,1 mm et 0,5 mm.

35. Dispositif selon l'une quelconque des revendications 33 à 34, dans lequel les moyens de positionnement et de guidage permettent le déplacement automatisé du masque (10) le long du support d'analyse.

36. Procédé de dépôt et d'étalement d'un ou plusieurs réactifs sur un support d'analyse comprenant des échantillons biologiques comprenant les étapes de :
- chargement du ou des réactifs sur le masque (10) de façon à permettre le dépôt du ou des réactifs sur le support d'analyse et leur maintien par capillarité entre ledit support et la pente de la ou des pistes dudit masque ;
- positionnement d'un masque (10) selon l'une quelconque des revendications 1 à 32 ou d'un dispositif selon l'une quelconque des revendications 33 à 35, à proximité du support d'analyse de façon à permettre le dépôt du ou des réactifs sur le support d'analyse et leur maintien par capillarité entre ledit support et la pente de la ou des pistes dudit masque ;
- déplacement du masque de façon à réaliser un balayage de la surface du support d'analyse pour permettre l'étalement du ou des réactifs sur le support d'analyse au niveau de zones d'incubation, correspondant à des zones délimitées dudit support, le ou les réactifs étant étalés en quantité suffisante pour permettre leur interaction avec les constituants des échantillons biologiques présents sur ledit support d'analyse.

37. Procédé selon la revendication 36 dans lequel le déplacement du masque permet le dépôt d'une quantité de chaque réactif comprise entre 4µl et 10µl par piste sur les zones délimitées du support d'analyse.

38. Procédé selon l'une quelconque des revendications 36 ou 37, dans lequel le chargement du masque au moyen du ou des réactifs s'effectue en dehors de la zone de la surface du support d'analyse comprenant les constituants des échantillons biologiques.

39. Procédé selon la revendication 36, dans lequel le chargement du masque au moyen du ou des réactifs, s'effectue préalablement à l'étape de positionnement du masque à proximité du support d'analyse et en ce que les réactifs sont déposés sur le support d'analyse à la suite d'une impulsion résultant d'une pression d'air exercée au niveau du masque, d'une jonction mécanique entre les réactifs et le support d'analyse, d'une projection des réactifs sur le support, ou d'un bref contact entre la masque et le support d'analyse, au niveau du point le plus bas de la pente de la piste.

40. Procédé selon la revendication 39, dans lequel le chargement du masque (10) au moyen du ou des réactifs est automatisé.

41. Procédé selon l'une quelconque des revendications 36 à 40, dans lequel l'étape de déplacement du masque (10) est automatisée.

42. Procédé selon l'une quelconque des revendications 36 à 41, dans lequel le support d'analyse est un support d'électrophorèse sur lequel ont été séparés par migration électrophorétique, les constituants d'un ou plusieurs échantillons biologiques.

43. Procédé selon la revendication 42, dans lequel le ou les réactifs sont destinés à permettre une réaction d'immunofixation des constituants des échantillons biologiques séparés par électrophorèse.

44. Procédé selon la revendication 43, dans lequel l'un des réactifs est un fixateur permettant de fixer tous les constituants séparés des échantillons biologiques par électrophorèse.

45. Procédé selon l'une des revendications 43 ou 44, dans lequel la réaction d'immunofixation inclut une étape de révélation des constituants des échantillons biologiques immunofixés.

46. Procédé selon la revendication 36, dans lequel le fixateur est chargé au niveau de la ou des pistes appropriées du masque puis déposé et étalé sur la ou les zones déterminées du support d'électrophorèse après l'étalement contrôlé du ou des réactifs sur le support d'analyse au niveau de zones délimitées dudit support, le fixateur et les réactifs étant étalés en quantité suffisante pour permettre leur réaction avec les constituants des échantillons biologiques présents sur ledit support d'analyse.

47. Procédé selon l'une quelconque des revendications 36 à 46, dans lequel la quantité de réactif chargée sur chaque piste du masque est égale à ou comprise entre 4 et 15 µl / piste.

48. Procédé selon l'une quelconque des revendications 36 à 47 dans lequel le masque destiné au dépôt et à l'étalement des réactifs est tel que la quantité de réactif chargée au niveau de chacune de ses pistes est en excès par rapport à la quantité de ce réactif étalée sur la zone d'incubation du
support d'électrophorèse lors d'un déplacement du masque de la position anodique à la position cathodique du support d'électrophorèse correspondant à la cathode.

49. Kit comprenant
- au moins un masque selon quelconque des revendications 1 à 32,
- au moins un support d'analyse.

50. Kit selon la revendication 49, comprenant en outre
- des réactifs pour l'immunofixation des constituants des échantillons séparés par électrophorèse,
- un fixateur pour la fixation pour chaque échantillon de l'ensemble des constituants séparés par électrophorèse.

51. Masque selon l'une quelconque des revendications 1 à 32 ou kit selon l'une quelconque des revendications 49 ou 50, dans lequel les réactifs sont chargés dans le masque et sont sous forme lyophilisée.

## Claims

1. A mask, suitable for depositing and distributing reagents on an analytical support for biological samples, comprising:
• a lower surface and an upper surface that are at least partially mutually parallel, separated by a distance constituting the thickness of the mask;
• one or more lane(s), corresponding to delimited zones, localized at the level of the lower surface of the mask and comprising a projecting element (32) that projects from the lower surface of the mask, each projecting element comprising a portion constituting a slope with respect to a horizontal plane;
• **characterized in that** the mask comprises, associated with each lane, an opening for loading and depositing reagents, said opening traversing the mask over the whole of its thickness from an upper loading orifice (36) on the upper surface of the mask to a lower deposit orifice (34), said lower deposit orifice being located in the slope, at proximity of the lowest point of the slope of the lane;
the mask being such that the lane or lanes it comprises can hold, by capillary action between the lane and the corresponding facing surface of the analytical support against which the mask is to be placed, reagents loaded into each opening and deposited on the analytical support.

2. A mask according to claim 1, suitable for distributing reagents on an analytical support for biological samples, comprising:
• a lower surface and an upper surface that are at least partially mutually parallel, separated by a distance constituting the thickness of the mask;
• one or more lane(s) each comprising a projecting element (32) of elongate shape emerging beneath the lower surface of the mask, said projecting element comprising a portion constituting a slope with respect to a horizontal plane;
• associated with each lane, an opening for loading and depositing reagents, said openingtraversing the mask over the whole of its thickness from an upper loading orifice (36) on the upper surface of the mask to a lower deposit orifice (34), said lower deposit orifice being located in the slope at proximity of the lowest point of the slope of the lane;
the mask being such that the lane or lanes it comprises can hold, by capillary action between the lane and the corresponding facing surface of the analytical support against which the mask is to be placed, reagents loaded into each opening and deposited on the analytical support.

3. A mask according to claim 1 or 2, suitable for distributing reagents on an analytical support for biological samples, comprising:
• a lower surface and an upper surface that are at least partially mutually parallel, separated by a distance constituting the thickness of the mask;
• one or more lane(s) each comprising a projecting element (32) emerging beneath the lower surface of the mask, constituted by a protuberance in the shape of a truncated parallelepiped, said projecting element comprising a portion constituting a slope with respect to a horizontal plane;
• associated with each lane, an opening for loading and depositing reagents, said opening traversing the mask over the whole of its thickness from an upper loading orifice (36) on the upper surface of the mask to a lower deposit orifice (34), said lower deposit orifice being located in the slope at the proximity of the lowest point of the slope of the lane;
the mask being such that the lane or lanes it comprises can hold, by capillary action between the lane and the corresponding facing surface of the analytical support against which the mask is to be placed, reagents loaded into each opening and deposited on the analytical support.

4. A mask according to claim 2 or 3, suitable for distributing reagents on an analytical support for biological samples, wherein the projecting element (32) has an upper surface that is coextensive with the portion of the lower surface of the mask beneath which said projecting element emerges, and a lower surface separated from the upper surface by at least one slope with respect to a horizontal plane.

5. A mask according to claim 1, wherein each projecting element (32) comprises a lower surface and an upper surface that are mutually parallel and parallel to the lower and upper surface of the mask, the slope of the lane being produced by inclining the mask with respect to the analytical support in the position of use.

6. A mask according to claim 5, wherein the projecting element (32) is of elongate shape, in particular is of parallelepiped shape.

7. A mask according to claim 4 or 6, **characterized in that** the lowest point of the slope of the lane, located at proximity of the lower orifice (34) of each lane opening, is the point that is the closest to the analytical support when brought in the position of use.

8. A mask according to any one of claims 1 to 7, **characterized in that** it is rigid or stiffened.

9. A mask according to any one of claims 1 to 8, in which the volume of the opening is such that it can constitute a reservoir for the loaded reagents.

10. A mask according to any one of claims 1 to 9, in which the opening for each lane traverses the thickness of the mask including the thickness of the projecting element (32) in a perpendicular manner, the opening comprising a portion in the shape of a truncated cone (38) terminated by a lower orifice (34) that is cylindrical in shape.

11. A mask according to any one of claims 1 to 10, comprising a plurality of mutually parallel lanes, distributed over the length of the mask.

12. A mask according to claim 11, comprising:
• a first series of mutually parallel lanes disposed in a first alignment;
• a second series of mutually parallel lanes that are parallel to the lanes of the first series, and forming a second alignment offset with respect to the first alignment.

13. A mask according to claim 11, in which the lanes are organised in several groups along the mask's length, each group being constituted by a lane disposed offset with respect to the other lanes aligned in-between themselves, the lanes being mutually parallel lanes.

14. A mask according to any one of claims 1 to 4 and 8 to 13, in which the length of the slope of each lane coincides with the length of that lane.

15. A mask according to any one of claims 1 to 4 and 8 to 13, in which the length of the slope of each lane extends over the length of that lane.

16. A mask according to any one of claims 1 to 15, wherein the lanes allow depositing each reagent in a quantity equal to or in the range 4 to 15 µl in each opening of the mask and holding said reagents between the lanes of the mask and the analytical support by capillary action, when the mask is brought at proximity of the analytical support.

17. A mask according to claim 16, for depositing each reagent in a quantity equal to or in the range 4 to 15 µl in each opening of the mask and holding said reagents between the lanes of the mask and the analytical support by capillary action, when the mask is brought to a distance from the analytical support of 2 mm or less from the point of the mask that is furthest from the analytical support.

18. A mask according to any one of claims 1 to 17, for depositing each reagent in a quantity equal to or in the range 4 to 15 µl in each opening of the mask and holding said reagents between the lanes of the mask and the analytical support by capillary action, when the mask is brought to a distance from the analytical support equal to or in the range 0.1 to 0.5 mm from the point of the mask that is closest to the analytical support.

19. A mask according to any one of claims 1 to 18, in which the lanes are separated from each other by a distance of 1.5 mm or more.

20. A mask according to claim 19, in which the distance between the lanes is of 2.5 mm.

21. A mask according to any one of claims 1 to 20, in which the dimensions of the lanes and their spacing are such that the reagents held by them between the mask and the analytical support by capillary action do not interact during deposition or distribution onto said analytical support.

22. A mask according to any one of claims 1 to 21, in which the lane width is 2.5 mm.

23. A mask according to any one of claims 1 to 22, in which the length of each lane is in the range 6 to 7 mm.

24. A mask according to any one of claims 1 to 23, in which the lane intended for the fixative is offset with respect to the neighbouring first lane by a distance of 5 to 7 mm.

25. A mask according to any one of claims 1 to 24, in which the slope of each lane forms an angle in the range 1 ° to 10° to a horizontal plane.

26. A mask according to any one of claims 1 to 25, in which each lane has the following dimensions:
• length: 3 to 15 mm;
• width: 1 to 10 mm;
• inclination of the slope: 1° to 10° to the horizontal.

27. A mask according to any one of claims 1 to 26, in which each lane has the following dimensions:
• length: 7 mm;
• width: 2,5 mm;
• inclination of the slope: 5° to the horizontal.

28. A mask according to any one of claims 1 to 27 in which the upper orifice of the opening has an angle of 50°.

29. A mask according to any one of claims 1 to 28 in which the thickness of the mask is comprised between 1 and 10 mm.

30. A mask according to any one of claims 1 to 29, comprising 6, 12 or 18 lanes.

31. A mask according to any one of claims 1 to 30, made in a material which is proper to be moulded, and allowing the making of a smooth surface.

32. A mask according to any one of claims 1 to 31, associated with positioning means intended to hold the mask in the proximity of the surface of the analytical support close to which the mask will be brought for deposition and distribution of reagents on the analytical support.

33. A device for depositing and distributing one or more reagents on an analytical support for biological samples, comprising:
a) a mask (10) according to any one of claims 1 to 32;
b) means (12, 14, 16) for positioning and guiding the mask allowing the mask to be positioned so that the mask is held in the proximity of the surface of the analytical support and allowing the mask to be guided by sweeping the surface of the analytical support in a horizontal plane parallel to the surface of said support to allow deposition and distribution of the reagents over each of the predetermined zones of the analytical support coming into line with the lanes of the mask.

34. A device according to claim 33, in which the means for positioning and guiding the mask (10) can establish a distance between the analytical support and the point on the mask that is closest to said support in the range 0.1 mm to 0.5 mm.

35. A device according to any one of claims 33 or 34, in which the positioning and guiding means allow automatic displacement of the mask (10) along the analytical support.

36. A method for depositing and distributing one or more reagents on an analytical support comprising biological samples, comprising the steps of:
• loading the reagent or reagents onto a mask (10) to allow the reagent or reagents to be deposited on the analytical support, and being held between said support and the slope or the lane or lanes of said mask by capillary action;
• positioning a mask (10) according to any one of claims 1 to 32 or a device according to any one of claims 33 to 35, at the proximity of the analytical support to deposit the reagent or reagents on the analytical support and hold them between said support and the slope of the lane or lanes of said mask by capillary action;
• displacing the mask by sweeping the surface of the analytical support to distribute the reagent or reagents on the analytical support in incubation zones, corresponding to delimited zones of said support, the reagent or reagents being distributed in a quantity sufficient to allow their interaction with the constituents of the biological samples present on said analytical support.

37. A method according to claim 36, in which sweeping of the mask allow the deposit of a quantity of each reagent that is comprised between 4 µl and 10µl by lane on the delimited zones of the analytical support.

38. A method according to any one of claim 36 or claim 37, in which the mask is loaded with the reagent or reagents away from the zone of the surface of the analytical support comprising the constituents of the biological samples.

39. A method according to claim 36, in which the mask is loaded with the reagent or reagents prior to the step for positioning the mask in the proximity of the analytical support and in that the reagents are deposited on the analytical support following an impulse resulting from air pressure exerted on the mask, or by a mechanical junction between the reagents and the analytical support, or by projecting the reagents onto the support, or by brief contact between the mask and the analytical support at the lowest point of the slope of the lane.

40. A method according to claim 39, in which loading of the mask (10) with the reagent or reagents is in an automated manner.

41. A method according to any one of claims 36 to 40, in which the displacement of the mask (10) is in an automated manner.

42. A method according to any one of claims 36 to 41, in which the analytical support is an electrophoresis support on which the constituents of one or more biological samples have been separated by electrophoretic migration.

43. A method according to claim 42, in which the reagent or reagents are intended to allow immunofixation of the constituents of biological samples separated by electrophoresis.

44. A method according to claim 43, in which one of the reagents is a fixative allowing fixing all the constituents of biological samples separated by electrophoresis.

45. A method according to any one of claims 43 or 44, in which the immunofixation reaction encompasses a step of revelation of the constituents of the immunofixated biological samples.

46. A method according to claim 36, in which the fixative is loaded into the appropriate lane or lanes of the mask, then deposited and distributed over the predetermined zone(s) of the electrophoretic support after controlled sweeping of the reagent(s) on the analytical support within the delimited zones of said support, the fixative and the reagents being distributed in a sufficient quantity to allow their reaction with the constituents of the biological samples found on said analytical support.

47. A method according to any one of claims 36 to 46, in which the quantity of reagent loaded onto each lane of the mask is equal to or in the range 4 to 15 µl in each lane.

48. A method according to any one of claims 36 to 47, in which the mask suitable for depositing and distributing reagents is constituted so that the quantity of reagent loaded onto each of its lanes is in excess by comparison with the quantity of said reagent distributed onto the incubation zone of the electrophorectic support when the mask is swept from the anodic position to the cathodic position of the electrophorectic support, corresponding to the cathode.

49. A kit comprising:
• at least one mask according to any one of claims 1 to 32;
• at least one analytical support.

50. A kit according to claim 49, further comprising:
• reagents for immunofixation of the constituents of samples separated by electrophoresis;
• a fixative for fixing each sample of the assembly of constituents separated by electrophoresis.

51. A mask according to any one of claims 1 to 32 or kit according to any one of claims 49 or 50, in which the reagents are loaded into the mask and are in the freeze dried form.

## Patentansprüche

1. Maske zur Auftragung und Verteilung von Reagenzien auf einen Träger zur Analyse biologischer Proben, umfassend:
- eine untere Oberfläche und eine obere Oberfläche, die mindestens teilweise parallel zueinander verlaufen und durch eine der Dicke der Maske entsprechende Entfernung beabstandet sind,
- eine oder mehrere Bahn(en), die einem oder mehreren abgegrenzten Bereiche(n) entsprechen und an der unteren Oberfläche der Maske angeordnet sind und ein vorstehendes Element (32) umfassen, das von der unteren Oberfläche des Maske hervorsteht, worin jedes vorstehende Element einen Bereich umfasst, der relativ zu einer horizontal verlaufenden Ebene eine Schräge aufweist,
- **dadurch gekennzeichnet, dass** die Maske eine jeweils einer Bahn zugeordnete Öffnung zur Beladung und zur Auftragung von Reagenzien umfasst, worin die Öffnung die Maske über deren gesamte Dicke ausgehend von einer oberen Beladungs-Öffnung (36) an der oberen Oberfläche der Maske bis zu einer unteren Auftragungs-Mündung (34) durchläuft, worin die untere Auftragungs-Mündung (34) in der Schräge nahe der untersten Stelle der Schräge der Bahn angeordnet ist,
worin die Maske derart ausgestaltet ist, dass die darin enthaltene(n) Bahn(en) ermöglichen, die aufgetragenen Reagenzien durch Kapillarwirkung zwischen der Bahn und der Oberfläche des Analyseträgers, an dem die Maske angelegt ist, auf dem Niveau jeder Öffnung aufrecht zu erhalten und diese auf dem Analyseträger aufzutragen.

2. Maske nach Anspruch 1 zur Verteilung von Reagenzien auf einen Träger zur Analyse biologischer Proben, umfassend:
- eine untere Oberfläche und eine obere Oberfläche, die mindestens teilweise parallel zueinander verlaufen und durch eine der Dicke der Maske entsprechende Entfernung beabstandet sind,
- eine oder mehrere Bahn(en), die jeweils ein längliches vorstehendes Element (32) umfassen, das unter der unteren Oberfläche der Maske hervorsteht, worin das vorstehende Element einen Bereich umfasst, der relativ zu einer horizontal verlaufenden Ebene eine Schräge aufweist,
- eine jeweils einer Bahn zugeordnete Öffnung zur Beladung und Auftragung von Reagenzien, worin die Öffnung die Maske über deren gesamte Dicke ausgehend von der oberen Beladungs-Öffnung (36) an der oberen Oberfläche der Maske bis zu einer unteren Auftragungs-Mündung (34) durchläuft, worin die untere Auftragungs-Öffnung in der Schräge, in der Nähe der untersten Stelle der Schräge der Bahn angeordnet ist,
worin die Maske derart ausgestaltet ist, dass die darin enthaltene(n) Bahn(en) ermöglichen, die aufgetragenen Reagenzien durch Kapillarwirkung zwischen der Bahn und der Oberfläche des Analyseträgers, an dem die Maske angelegt ist, auf dem Niveau jeder Öffnung aufrecht zu erhalten und diese auf dem Analyseträger aufzutragen.

3. Maske nach Anspruch 1 oder 2, zur Verteilung von Reagenzien auf einen Träger zur Analyse biologischer Proben, umfassend:
- eine untere Oberfläche und eine obere Oberfläche, die mindestens teilweise parallel zueinander verlaufen und durch eine der Dicke der Maske entsprechende Entfernung beabstandet sind,
- eine oder mehrere Bahn(en), die jeweils ein vorstehendes Element (32) umfassen, das unter der unteren Oberfläche des Maske hervorsteht, worin das Element durch eine abgestumpften quaderförmigen Vorsprung ausgebildet ist, worin das vorstehende Element einen Bereich umfasst, der relativ zu einer horizontal verlaufenden Ebene eine Schräge aufweist,
- eine Öffnung, die jeweils einer Bahn zugeordnet ist, zur Beladung und Verteilung der Reagenzien, worin die Öffnung die Maske über deren gesamte Dicke ausgehend von einer oberen Beladungs-Öffnung (36) an der oberen Oberfläche der Maske bis zu einer unteren Auftragungs-Mündung (34) durchläuft, worin die untere Auftragungs-Öffnung in der Schräge, in der Nähe der untersten Stelle der Schräge der Bahn angeordnet ist,
worin die Maske derart ausgestaltet ist, dass die darin enthaltene(n) Bahn(en) ermöglichen, die aufgetragenen Reagenzien durch Kapillarwirkung zwischen der Bahn und der Oberfläche des Analyseträgers, an dem die Maske angelegt ist, auf dem Niveau jeder Öffnung aufrecht zu erhalten und diese auf dem Analyseträger aufzutragen.

4. Maske nach Anspruch 2 oder 3, zur Verteilung von Reagenzien auf einen Träger zur Analyse biologischer Proben, **dadurch gekennzeichnet, dass** das vorstehende Element (32) mit einer oberen Oberfläche, die mit dem Bereich der unteren Oberfläche der Maske, von der es hervorsteht, übereinstimmt, und dass eine untere Oberfläche von der oberen Oberfläche durch mindestens eine Schräge relativ zu einer waagerechten Ebene beabstandet ist.

5. Maske nach Anspruch 1, **dadurch gekennzeichnet, dass** jedes vorstehende Element (32) eine untere Oberfläche und eine obere Oberfläche umfasst, die parallel zueinander und parallel zu den unteren und oberen Oberflächen der Maske vorliegen, worin die Schräge der Bahn durch Neigung der Maske im Verhältnis zum Analyseträger bei Verwendungsposition erfolgt.

6. Maske nach Anspruch 5, **dadurch gekennzeichnet, dass** das vorstehende Element (32) länglich, insbesondere quaderförmig ist.

7. Maske nach Anspruch 4 oder 6, **dadurch gekennzeichnet, dass** die unterste Stelle der Schräge, die in der Nähe der unteren Mündung (34) der Öffnung jeder Bahn angeordnet ist, die dem Analyseträger nächstliegende Stelle ist, und in seiner Nähe die Einsatzstellung ist.

8. Maske nach einem der Ansprüche 1 bis 7, **dadurch gekennzeichnet, dass** sie steif oder versteift ist.

9. Maske nach einem der Ansprüche 1 bis 8, worin die Öffnung ein Volumen aufweist, so dass es für die beladenen Reagenzien einen Speicher ausbildet.

10. Maske nach einem der Ansprüche 1 bis 9, worin die Öffnung jeder Bahn die Dicke der Maske, einschließlich der Dicke des vorstehenden Elements (32) senkrecht durchquert, wobei die Öffnung einen kegelstumpfartigen Teil (38) aufweist, der mit einer unteren, zylinderförmigen Mündung (34) endet.

11. Maske nach einem der Ansprüche 1 bis 10, umfassend mehrere zueinander parallele Bahnen, die auf die Länge der Maske verteilt sind.

12. Maske nach Anspruch 11, umfassend:
- eine erste Reihe von zueinander parallel angeordneten Bahnen, die in einer ersten Ausrichtung angeordnet sind,
- eine zweite Reihe von zueinander und zu den Bahnen der ersten Reihe parallel angeordneten Bahnen, die eine zweite Ausrichtung ausbilden, die gegenüber der ersten Ausrichtung versetzt ist.

13. Maske nach Anspruch 11, worin die Bahnen in mehreren, über die Länge der Maske hintereinander angeordneten Gruppen angeordnet sind, worin jede Gruppe aus einer Bahn besteht, die im Verhältnis zu den anderen zueinander ausgerichteten Bahnen versetzt angeordnet ist, worin die Bahnen parallel zueinander vorliegen.

14. Maske nach einem der Ansprüche 1 bis 4 und 8 bis 13, worin die Länge der Schräge jeder Bahn mit der Länge der Bahn übereinstimmt.

15. Maske nach einem der Ansprüche 1 bis 4 und 8 bis 13, worin die Bahn über die Schräge hinaus reicht.

16. Maske nach einem der Ansprüche 1 bis 15, worin die Bahnen die Auftragung eines jeden Reagenz in einer Menge, die gleich oder zwischen 4 und 15 µl liegt, in der Öffnung der Maske ermöglichen und die Aufrechterhaltung durch Kapillarwirkung dieser Reagenzien zwischen der Bahn und dem Analyseträger ermöglichen, wenn die Maske in die Nähe des Analyseträgers gebracht wird.

17. Maske nach Anspruch 16, welche eine Auftragung eines jeden Reagenz in einer Menge, die gleich oder zwischen 4 und 15 µl liegt, in jeder Öffnung der Maske ermöglicht und eine Aufrechterhaltung durch Kapillarwirkung der Reagenzien zwischen der Bahn und des Analyseträgers, wenn die Maske in einen Abstand vom Analyseträger, der weniger oder gleich 2 mm ist, zur entferntesten Stelle des Analyseträgers gebracht wird.

18. Maske nach einem der Ansprüche 1 bis 17, welche eine Auftragung eines jede Reagenz in einer Menge, die gleich oder zwischen 4 und 15 µl liegt, in jeder Öffnung der Maske ermöglicht und eine Aufrechterhaltung durch Kapillarwirkung dieser Reagenzien zwischen den Bahnen der Maske und dem Analyseträger ermöglicht, wenn die Maske in einen Abstand vom Analyseträger, der gleich oder zwischen 0,1 und 0,5 mm liegt, zur nächstliegenden Stelle des Analyseträgers gebracht wird.

19. Maske nach einem der Ansprüche 1 bis 18, worin die Bahnen durch einen Abstand, der größer oder gleich 1,5 mm ist, voneinander entfernt sind.

20. Maske nach Anspruch 19, worin der Abstand zwischen den Bahnen 2,5 mm beträgt.

21. Maske nach einem der Ansprüche 1 bis 20, worin die Abmessungen der Bahnen und deren Abstand derart sind, dass die so zwischen der Maske und dem Analyseträger durch Kapillarwirkung aufrechterhaltenen Reagenzien bei der Auftragung oder der Verteilung auf dem Analyseträger nicht miteinander reagieren.

22. Maske nach einem der Ansprüche 1 bis 21, worin die Breite der Bahnen 2,5 mm beträgt.

23. Maske nach einem der Ansprüche 1 bis 22, worin jede Bahn eine Länge zwischen 6 und 7 mm aufweist.

24. Maske nach einem der Ansprüche 1 bis 23, worin die für das Fixativ ausgelegte Bahn relativ zur ersten benachbarten Bahn um eine Entfernung von 5 bis 7 mm versetzt ist.

25. Maske nach einem der Ansprüche 1 bis 24, worin die Schräge einer jeden Bahn mit einer waagerechten Ebene einen Winkel zwischen 1° und 10° ausbildet.

26. Maske nach einem der Ansprüche 1 bis 25, worin jede Bahn die folgenden Abmessungen aufweist:
- Länge: 3 bis 15 mm
- Breite: 1 bis 10 mm
- Neigung der Schräge relativ zu einer waagerechten Ebene: 1 bis 10°.

27. Maske nach einem der Ansprüche 1 bis 26, worin jede Bahn die folgenden Abmessungen aufweist:
- Länge: 7 mm.
- Breite: 2,5 mm.
- Winkel der Schräge relativ zu einer waagerechten Ebene: 5°.

28. Maske nach einem der Ansprüche 1 bis 27, in welcher die obere Mündung der Öffnung einen Winkel von 50° ausbildet.

29. Maske nach einem der Ansprüche 1 bis 28, worin die Dicke der Maske zwischen 1 und 10 mm beträgt.

30. Maske nach einem der Ansprüche 1 bis 29, umfassend 6, 12 oder 18 Bahnen.

31. Maske nach einem der Ansprüche 1 bis 30, aus einem formbaren Material und zur Erzeugung einer glatten Oberfläche.

32. Maske nach einem der Ansprüche 1 bis 31, die zur Aufrecherhaltung der Maske in der Nähe der Oberfläche des Analyseträgers mit Positionierungsmitteln ausgestattet ist, wobei zur Auftragung und Verteilung von Reagenzien auf dem Analyseträger die Maske in die Nachbarschaft dieses Trägers überführt wird.

33. Einrichtung zur Auftragung und Verteilung einer oder mehrerer Reagenzien auf einem Analyseträger für biologische Proben, umfassend:
a) eine Maske (10) nach einem der Ansprüche 1 bis 32,
b) Mittel (12, 14, 16) zur Positionierung und Führung der Maske, die eine Positionierung der Maske ermöglichen, derart, dass die Maske in der Nähe der Oberfläche des Analyseträgers gehalten wird und eine Führung der Maske zum Abtasten der Oberfläche des Analyseträgers in einer waagerechten Ebene parallel zur Oberfläche des Trägers oder geneigt relativ zur Oberfläche dieses Trägers ermöglicht wird, ist, um die Auftragung und die Verteilung der Reagenzien auf jedem der bestimmten Bereiche des den Bahnen der Maske gegenüberliegenden Analyseträgers zu ermöglichen.

34. Einrichtung nach Anspruch 33, worin die Mittel zur Positionierung und Führung der Maske (10) die Ermittlung einer Entfernung, die zwischen 0,1 und 0,5 mm beträgt, zwischen dem Analyseträger und der Stelle der Bahn der nächstliegenden Maske dieses Trägers erlauben.

35. Einrichtung nach einem der Ansprüche 33 bis 34, worin die Mittel zur Positionierung und Führung die automatisierte Bewegung der Maske (10) entlang des Analyseträgers erlauben.

36. Verfahren zum Auftragen und zur Verteilung einer oder mehrerer Reagenzien auf einen Analyseträger mit biologischen Proben, das die folgenden Schritte umfasst:
- Auftragen des Reagenzes oder der Reagenzien auf die Maske (10), um die Auftragung des Reagenzes oder der Reagenzien auf dem Analyseträger zu ermöglichen und deren Aufrechterhaltung durch Kapillarwirkung zwischen dem Träger und der Schräge der Bahn(en) dieser Maske zu ermöglichen;
- Positionieren einer Maske (10) nach einem der Ansprüche 1 bis 32 oder einer Einrichtung nach einem der Ansprüche 33 bis 35 in der Nähe des Analyseträgers, um die Auftragung des Reagenzes oder der Reagenzien auf dem Analyseträger zu ermöglichen und deren Aufrechterhaltung durch Kapillarwirkung zwischen diesem Träger und der Schräge der Bahn(en) dieser Maske zu ermöglichen;
- Bewegen der Maske, um eine Abtastung der Oberfläche des Analyseträgers durchzuführen, um das Reagenz oder die Reagenzien auf dem Analyseträger in Inkubationsbereichen zu verteilen, die abgegrenzten Bereichen dieses Trägers entsprechen, wobei das Reagenz oder die Reagenzien in ausreichender Menge verteilt wird/werden, um mit den Komponenten der auf diesem Analyseträger vorhandenen biologischen Proben zusammenzuwirken.

37. Verfahren nach Anspruch 36, wobei die Bewegung der Maske die Auftragung einer Menge eines jeden Reagenz auf den abgegrenzten Bereichen des Analyseträgers zwischen 4µl und 10µl pro Bahn erlaubt.

38. Verfahren nach einem der Ansprüche 36 bis 37, wobei die Auftragung des Reagenzes oder der Reagenzien auf die Maske ausserhalb des Bereichs der Oberfläche des die Komponenten der biologischen Proben enthaltenden Analyseträgers erfolgt.

39. Verfahren nach Anspruch 36, wobei die Auftragung des Reagenzes oder der Reagenzien auf die Maske vor der Stufe der Positionierung der Maske in der Nähe des Analyseträgers erfolgt und wobei die Reagenzien auf dem Analyseträger nach einem Impuls aufgetragen werden, der durch Anlegen von Druckluft auf die Maske, einer mechanischen Verbindung zwischen den Reagenzien und dem Analyseträger, einer Projizierung der Reagenzien auf den Träger oder einem kurzen Kontakt zwischen der Maske und dem Analyseträger bei der untersten Stelle der Schräge der Bahn erzeugt wird.

40. Verfahren nach Anspruch 39, wobei das das Auftragen des Reagenzes oder der Reagenzien auf die Maske (10) automatisiert ist.

41. Verfahren nach einem der Ansprüche 36 bis 40, wobei Stufe der Bewegung der Maske (10) automatisiert ist.

42. Verfahren nach einem der Ansprüche 36 bis 41, wobei der Analyseträger ein Elektrophorese-Träger ist, auf welchem die Komponenten eines oder mehrerer biologischer Proben durch elektrophoretische Migration getrennt worden sind.

43. Verfahren nach Anspruch 42, wobei das Reagenz oder die Reagenzien ausgelegt ist/sind, um eine Immunfixationsreaktion der Komponenten der durch Elektrophorese aufgetrennten biologischen Proben zu ermöglichen.

44. Verfahren nach Anspruch 43, wobei eines der Reagenzien ein Fixativ ist, das eine Fixierung aller Komponenten der durch Elektrophorese aufgetrennten biologischen Proben ermöglicht.

45. Verfahren nach einem der Ansprüche 43 oder 44, wobei die Immunfixationsreaktion eine Stufe des Nachweises der Komponenten der immunfixierten biologischen Proben umfasst.

46. Verfahren nach Anspruch 36, wobei das Fixativ bei der oder den jeweiligen Bahnen der Maske aufgetragen wird, anschließend auf dem oder den bestimmten Bereich(en) des Elektrophorese-Trägers aufgetragen und verteilt wird, wobei nach der gesteuerten Verteilung des Reagenzes oder der Reagenzien auf dem Analyseträger auf die abgegrenzten Bereiche des Trägers das Fixativ und die Reagenzien in ausreichender Menge verteilt werden, um deren Reaktion mit den Komponenten der auf diesem Analyseträger vorhandenen biologischen Proben zu ermöglichen.

47. Verfahren nach einem der Ansprüche 36 bis 46, wobei die auf jeder Bahn der Maske zugegebene Reagenzmenge gleich oder zwischen 4 und 15 µl/Bahn liegt.

48. Verfahren nach einem der Ansprüche 36 bis 47, wobei die Maske zur Auftragung und Verteilung der Reagenzien derart ist, dass die bei jeder dieser Bahnen zugegebene Reagenzmenge gegenüber der Menge dieses Reagenzes, die auf dem Inkubationsbereich des Elektrophorese-Trägers bei der Bewegung der Maske von der Anoden-Position zur Kathoden-Position, die dem Elektrophorese-Träger entspricht, verteilt wird, überschüssig ist.

49. Kit umfassend:
- mindestens eine Maske nach einem der Ansprüche 1 bis 32,
- mindestens einen Analyseträger.

50. Kit nach Anspruch 49, weiter umfassend:
- Reagenzien zur Immunfixierung der Komponenten der durch Elektrophorese aufgetrennten Proben,
- ein Fixativ zur Fixierung für jede Probe der durch Elektrophorese getrennten Komponenten.

51. Maske nach einem der Ansprüche 1 bis 32, oder Kit nach einem der Ansprüche 49 oder 50, worin die Reagenzien in die Maske zugegeben werden und in lyophilisierter Form vorliegen.
